# EUROPÄISCHE PATENTSCHRIFT

(11) **EP 4 209 242 B1**
(45) Veröffentlichungstag und Bekanntmachung des Hinweises auf die Patenterteilung: **09.10.2024**
(21) Anmeldenummer: 22212814.2
(22) Anmeldetag: 12.12.2022
(51) Int. Cl.: A61M 16/08

(54) **ABSORPTIONS-ANORDNUNG MIT EINEM CO2-ABSORBER UND EINER WASSERFALLE UND VERFAHREN ZUM HERAUSFILTERN VON CO2**
ABSORPTION ASSEMBLY COMPRISING A CO2 ABSORBER AND A WATER TRAP AND METHOD FOR FILTERING OUT CO2
ENSEMBLE D'ABSORPTION COMPRENANT UN ABSORBEUR DE CO2 ET UN PIÈGE À EAU ET PROCÉDÉ D'EXTRACTION DE CO2

(30) Priorität: 07.01.2022 DE 102022100286
(43) Veröffentlichungstag der Anmeldung: 12.07.2023
(73) Patentinhaber: Drägerwerk AG & Co. KGaA, 23558 Lübeck (DE)
(72) Erfinder: PRIESKE, Marcel, 23558 Lübeck (DE); SCHNAARS, Henryk, 23558 Lübeck (DE)

(56) Entgegenhaltungen:
- EP-B1- 1 230 943
- US-A- 4 502 876
- US-A1- 2007 051 367
- US-A1- 2010 199 993
- US-B2- 7 850 765

## Beschreibung

Die Erfindung betrifft eine Absorptions-Anordnung umfassend einen CO2-Absorber und eine Wasserfalle sowie ein Verfahren, um unter Verwendung einer solchen Absorptions-Anordnung durch Absorption Kohlendioxid aus einem Gasgemisch herauszufiltern.

Die Aufgabe, aus einem Gasgemisch Kohlendioxid herauszufiltern, tritt beispielsweise bei der künstlichen Beatmung eines Patienten auf. Der Patient wird mit einem Gasgemisch versorgt, wobei dieses Gasgemisch Sauerstoff und optional ein Narkosemittel umfasst. Das vom Patienten ausgeatmete Gasgemisch enthält bekanntlich Kohlendioxid (CO2) und fließt zurück zum Beatmungsgerät. Aus diesem Gasgemisch soll das Kohlendioxid herausgefiltert werden, bevor das Gasgemisch wieder zum Patienten gefördert wird.

US 4,502,876 zeigt eine Absorptions-Anordnung, die sich mit einem Beatmungsgerät (rebreathing apparatus) verbinden lässt. Das Gasgemisch fließt durch eine Röhre 32 hindurch nach unten in eine perforierte Hülse 20 und aus der Hülse 20 nach außen in einen Filter 13, der ein CO2-absorbierendes Material 24 aufweist. Das Gasgemisch tritt durch Öffnungen in der Mantelfläche des Filters 13 aus und gelangt in einen röhrenförmigen Spalt zwischen dem Filter 13 und einer metallischen Röhre 14. Das Gasgemisch fließt in diesem Spalt nach unten und gelangt in einen inneren Beutel (flexible container 15) und von dort weiter nach unten in einen äußeren Beutel (flexible container 16). Das Gasgemisch fließt aus dem äußeren Beutel 16 nach oben in einen Raum oberhalb des Filters 13 und weiter nach oben durch eine Röhre 31 hindurch. Die Röhre 31 umgibt die Röhre 32. Kondensierte Flüssigkeit setzt sich an der Röhre 14 ab und tropft nach unten in den äußeren Beutel 16.

Der Erfindung liegt die Aufgabe zugrunde, eine Absorptions-Anordnung mit einem CO2-Absorber und ein Verfahren bereitzustellen, welche Kohlendioxid aus einem durch den CO2-Absorber fließenden Gasgemisch zu absorbieren vermögen und eine bessere Wirkung als bekannte Absorptions-Anordnungen und Verfahren aufweisen.

Die Aufgabe wird durch eine Absorptions-Anordnung mit den Merkmalen des Anspruchs 1 und durch ein Verfahren mit den Merkmalen des Anspruchs 15 gelöst. Vorteilhafte Ausgestaltungen sind in den Unteransprüchen angegeben. Vorteilhafte Ausgestaltungen der erfindungsgemäßen Absorptions-Anordnung sind, soweit sinnvoll, auch vorteilhafte Ausgestaltung des erfindungsgemäßen Verfahrens und umgekehrt.

Die erfindungsgemäße Absorptions-Anordnung umfasst eine Zuführ-Fluidführungseinheit und eine Abführ-Fluidführungseinheit. Die Zuführ-Fluidführungseinheit ist wenigstens zeitweise mit einer Quelle für ein Gasgemisch verbunden oder lässt sich mit einer solchen Quelle verbinden. Die Abführ-Fluidführungseinheit ist wenigstens zeitweise mit einer Senke für ein Gasgemisch verbunden oder lässt sich mit einer solchen Senke verbinden. In einer Realisierungsform lässt die Zuführ-Fluidführungseinheit sich mit einer quellenseitigen Koppeleinheit verbinden, wobei die quellenseitige Koppeleinheit in einer Fluidverbindung mit der Quelle steht. Die Abführ-Fluidführungseinheit lässt sich mit einer senkenseitigen Koppeleinheit verbinden, wobei die senkenseitige Koppeleinheit in einer Fluidverbindung mit der Senke steht.

Unter einer "Fluidführungseinheit" wird ein Bauteil verstanden, welches ein Fluid, insbesondere ein Gas oder eine Flüssigkeit, entlang einer vorgegebenen Bahn (Trajektorie) zu führen vermag. Dieses Bauteil verhindert vollständig oder wenigstens weitgehend, dass das Fluid diese Bahn verlässt. Die Fluidführungseinheit umfasst zwei Öffnungen, durch welche ein Fluid in die Fluidführungseinheit hineinfließen und / oder aus der Fluidführungseinheit austreten kann. Die Fluidführungseinheit kann insbesondere als eine starre Röhre oder als ein biegsamer Schlauch ausgestaltet sein oder eine Röhre und / oder einen Schlauch umfassen, auch einen Zwei-Lumen-Schlauch.

Zwischen zwei Bauteilen ist eine Fluidverbindung hergestellt, wenn ein Fluid von dem einen Bauteil zu dem anderen Bauteil fließen kann. Möglich ist, dass das Fluid direkt von dem einen Bauteil zu dem anderen Bauteil fließen kann. Möglich ist auch, dass die beiden Bauteile voneinander beabstandet sind und eine Fluidführungseinheit die Fluid Verbindung herstellt.

Die erfindungsgemäße Absorptions-Anordnung umfasst weiterhin einen CO2-Absorber und eine Wasserfalle. Der CO2-Absorber vermag Kohlendioxid (CO2) zu absorbieren und dadurch aus einem Gasgemisch, welches durch den CO2-Absorber strömt, Kohlendioxid herauszufiltern. Anders formuliert: Der Gehalt von Kohlendioxid in einem Gasgemisch, welches durch den CO2-Absorber strömt, ist nach dem Austritt aus dem CO2-Absorber kleiner als beim Eintritt in den CO2-Absorber. Idealerweise enthält das austretende Gasgemisch überhaupt kein Kohlendioxid mehr. Die Wasserfalle vermag eine Feuchtigkeit aufzunehmen, insbesondere Wasser. Die Wasserfalle umfasst einen entsprechenden Behälter.

Weiterhin umfasst die erfindungsgemäße Absorptions-Anordnung eine Verbindungs-Fluidführungseinheit. Die Verbindungs-Fluidführungseinheit verbindet den CO2-Absorber mit der Wasserfalle.

Außerdem umfasst die erfindungsgemäße Absorptions-Anordnung eine untere Umlenk-Fluidführungseinheit und eine obere Umlenk-Fluidführungseinheit. Jede Umlenk-Fluidführungseinheit vermag die Richtung eines Gasgemischs zu ändern, welches durch die Umlenk-Fluidführungseinheit fließt, bevorzugt um einen Winkel von mindestens 90 Grad, besonders bevorzugt von mindestens von 150 Grad. Die Begriffe "oben" und "unten" beziehen sich auf eine Orientierung bei einem produktiven Einsatz der Absorptions-Anordnung. Die obere Umlenk-Fluidführungseinheit befindet sich beim Einsatz senkrecht oder schräg oberhalb der unteren Umlenk-Fluidführungseinheit.

Die erfindungsgemäße Absorptions-Anordnung ist so ausgestattet, dass ein Gasgemisch, welches aus der Quelle zur Senke fließt, bei einem produktiven Einsatz auf dem folgenden Weg durch die Absorptions-Anordnung hindurch gezwungen wird:
- zuerst durch die Zuführ-Fluidführungseinheit,
- dann durch die untere Umlenk-Fluidführungseinheit,
- dann senkrecht oder schräg nach oben durch den CO2-Absorber,
- dann durch die obere Umlenk-Fluidführungseinheit,
- dann senkrecht oder schräg nach unten durch die Verbindungs-Fluidführungseinheit,
- dann durch die Wasserfalle und
- dann durch die Abführ-Fluidführungseinheit hindurch.

Natürlich ist es möglich, dass eine relativ kleine Menge des Gasgemischs aufgrund von unvermeidlichen Spalten und sonstigen Undichtigkeiten diesen Weg verlässt.

Die Begriffe "senkrecht oder schräg nach unten" und "senkrecht oder schräg nach oben" bezeichnen eine Fließrichtung, die von der Vertikalen um höchstens 60 Grad, bevorzugt um höchstens 45 Grad, besonders bevorzugt um höchstens 30 Grad abweicht. Eine solche Fließrichtung wird bei einem produktiven Einsatz der erfindungsgemäßen Absorptions-Anordnung erzielt. Außerhalb eines solchen produktiven Einsatzes kann die Absorptions-Anordnung anders positioniert sein, und daher können Fluidführungseinheiten anders im Raum orientiert sein.

Die erfindungsgemäße Absorptions-Anordnung umfasst einen CO2-Absorber und eine Wasserfalle. In vielen Fällen erspart die Erfindung die Notwendigkeit, zwei verschiedene und räumlich voneinander getrennte Bauteile vorsehen zu müssen, nämlich einen CO2-Absorber und eine davon getrennte Wasserfalle. Diese beiden voneinander getrennten Bauteile würden in vielen Fällen mehr Platz und / oder mehr und / oder längere Fluidführungseinheiten erfordern. Außerdem wäre es in vielen Fällen erforderlich, zwei Bauteile an zwei verschiedenen Stellen zu überwachen und bei Bedarf auszutauschen. Dank der Erfindung ist es in vielen Fällen möglich, ein einziges Bauteil auszutauschen und dadurch zu erneuern.

Erfindungsgemäß wird das Gasgemisch auf seinem Weg von der Quelle zur Senke zunächst von der unteren und dann von der oberen Umlenk-Fluidführungseinheit umgelenkt. Auf dem Weg von der unteren zur oberen Umlenk-Fluidführungseinheit fließt das Gasgemisch durch den CO2-Absorber hindurch. Die Konstruktion der Absorptions-Anordnung bewirkt in vielen Fällen, dass das Gasgemisch durch den gesamten CO2-Absorber hindurchfließt und nicht den CO2-Absorber an einer Stelle zwischen der unteren und der oberen Umlenk-Fluidführungseinheit verlassen kann. Dadurch wird sichergestellt, dass ein Absorptionsmaterial im CO2-Absorber vollständig ausgenutzt wird, bevor der CO2-Absorber ausgetauscht werden muss. Selbst wenn ein Bereich im Inneren des CO2-Absorbers vollständig gesättigt ist und kein weiteres Kohlendioxid mehr aufnehmen kann, vermag ein anderer Bereich noch Kohlendioxid aufzunehmen, es sei denn, der CO2-Absorber ist völlig verbraucht und muss ausgetauscht werden. In vielen Fällen verlängert diese Ausgestaltung daher die Lebensdauer des CO2-Absorbers verglichen mit anderen möglichen Ausgestaltungen. Der CO2-Absorber braucht seltener ausgetauscht zu werden.

Die erfindungsgemäße Absorptions-Anordnung bewirkt, dass das Gasgemisch senkrecht oder schräg nach oben durch die den CO2-Absorber fließt. Die Erfinder haben in internen Versuchen festgestellt, dass dieses Merkmal zu einer höheren Absorptionswirkung als eine andere mögliche Durchflussrichtung durch den CO2-Absorber aufgrund einer anderen Positionierung des CO2-Absorbers im Raum führt. Genauer gesagt: Ein größerer Anteil des Kohlendioxids, idealerweise das gesamte Kohlendioxid, wird aus dem durchströmenden Gasgemisch herausgefiltert. Bei einer anderen Durchflussrichtung oder Positionierung wird häufig ein geringerer Anteil herausgefiltert.

Diese Wirkung wird durch eine bevorzugte Ausgestaltung weiter vergrößert, bei der sich die untere Umlenk-Fluidführungseinheit bei einem produktiven Einsatz senkrecht oder schräg unterhalb des CO2-Absorbers befindet und der CO2-Absorber sich senkrecht oder schräg unterhalb der oberen Umlenk-Fluidführungseinheit befindet. Diese Wirkung wird außerdem weiter durch eine bevorzugte Realisierungsform des CO2-Absorbers vergrößert. Gemäß dieser Realisierungsform umfasst der CO2-Absorber ein Absorptionsmaterial, bevorzugt ein schuttfähiges Absorptionsmaterial, welches bevorzugt Aktivkohle umfasst, und ein eigenes Gehäuse um das Absorptionsmaterial herum.

Erfindungsgemäß fließt das Gasgemisch vom CO2-Absorber senkrecht oder schräg nach unten zur Wasserfalle. Dadurch fließen auch Flüssigkeitströpfchen, die im Gasgemisch enthalten sein oder sich durch Kondensation bilden können, nach unten und verbleiben in der Wasserfalle. In vielen Fällen bewirkt die Erfindung daher, dass sich im Gasgemisch flussabwärts von der Wasserfalle keine Flüssigkeitstropfen mehr befinden.

Bevorzugt fließt das Gasgemisch senkrecht oder schräg nach unten durch die Zuführ-Fluidführungseinheit oder wenigstens senkrecht oder schräg nach unten durch einen Abschnitt der Zuführ-Fluidführungseinheit. Diese Ausgestaltung erhöht die Zuverlässigkeit, dass das Gasgemisch tatsächlich zur unteren Umlenk-Fluidführungseinheit fließt und kein unerwünschter Rückfluss oder Rückstau in die entgegengesetzte Richtung auftritt. In vielen Fällen ist nämlich das Gasgemisch schwerer als Umgebungsluft, beispielsweise wenn es zusätzlich Narkosemittel enthält.

In vielen Fällen findet im CO2-Absorber eine exothermische chemische Reaktion statt, während ein Gasgemisch durch den CO2-Absorber strömt. Durch diese chemische Reaktion bindet der CO2-Absorber Kohlendioxid im Gasgemisch. Bei der chemischen Reaktion wird Wärme freigesetzt. In der Regel erwärmt sich dadurch das Gasgemisch, während es durch den CO2-Absorber fließt.

Verschiedene Ausgestaltungen sind möglich, um das Gasgemisch wieder zu kühlen. Nachfolgend werden einige bevorzugte Ausgestaltung beschrieben, um diese Wirkung zu erzielen.

Das Gasgemisch fließt vom CO2-Absorber durch die obere Umlenk-Fluidführungseinheit und die Verbindungs-Fluidführungseinheit hindurch zur Wasserfalle. Insbesondere dann, wenn die chemische Reaktion im CO2-Absorber Wärme freisetzt, ist das Gasgemisch flussabwärts vom CO2-Absorber In der Regel wärmer als die Umgebungsluft. In einer bevorzugten Ausgestaltung grenzt die Verbindungs-Fluidführungseinheit - oder wenigstens ein Abschnitt der Verbindungs-Fluidführungseinheit - unmittelbar an eine Umgebung der Absorptions-Anordnung an. Möglich ist auch, dass die obere Umlenk-Fluidführungseinheit - oder wenigstens ein Abschnitt der oberen Umlenk-Fluidführungseinheit - unmittelbar an die Umgebung angrenzt.

Das Merkmal, dass eine Fluidführungseinheit unmittelbar an die Umgebung angrenzt, bedeutet, dass ausschließlich die Wand dieser Fluidführungseinheit das durchströmende Gasgemisch von der Umgebung trennt, aber nicht eine weitere Wand, die von der Wand der Fluidführungseinheit beabstandet ist. Eine geeignete Ausgestaltung dieser Wand ermöglicht es, dass die Wand zwar das durchfließende Gasgemisch fluiddicht von der Umgebung trennt, jedoch einen großflächigen und dadurch guten thermischen Kontakt zwischen dem Gasgemisch, das durch die Fluidführungseinheit fließt, und der Umgebung herstellt. Wenn das Gasgemisch wärmer als die Umgebung ist, gibt das Gasgemisch durch die Wand hindurch Wärme an die Umgebung ab und kühlt sich dadurch ab.

Erfindungsgemäß fließt das Gasgemisch durch die Zuführ-Fluidführungseinheit und später durch den CO2-Absorber. In einer Ausgestaltung umgibt der CO2-Absorber die Zuführ-Fluidführungseinheit - oder wenigstens einen Abschnitt der Zuführ-Fluidführungseinheit - nach Art einer Ummantelung. In einer Realisierungsform bildet die Wand der Zufuhr-Fluidführungseinheit zugleich eine innere Wand für eine Kammer, welche Absorptionsmaterial aufnimmt, wobei diese Kammer zum CO2-Absorber gehört.

Das Merkmal, dass der CO2-Absorber die Zuführ-Fluidführungseinheit umgibt, bewirkt in vielen Fällen, dass ein großflächiger und dadurch guter thermischer Kontakt zwischen dem CO2-Absorber und der Zufuhr-Fluidführungseinheit hergestellt ist. Dadurch kann der CO2-Absorber Wärme an Gasgemische in der Zufuhr-Fluidführungseinheit abgeben. Das Gasgemisch fließt durch die Zufuhr-Fluidführungseinheit und anschließend durch die untere Umlenk-Fluidführungseinheit, bevor es in den CO2-Absorber eintritt. Ermöglicht wird eine Ausgestaltung, bei der die untere Umlenk-Fluidführungseinheit Wärme an die Umgebung abgibt.

Erfindungsgemäß fließt das Gasgemisch vom CO2-Absorber durch die obere Umlenk-Fluidführungseinheit und die Verbindungs-Fluidführungseinheit hindurch zur Wasserfalle. In einer Ausgestaltung umgibt die Verbindungs-Fluidführungseinheit - oder wenigstens ein Abschnitt der Verbindungs-Fluidführungseinheit - den CO2-Absorber nach Art einer Ummantelung. In einer Realisierungsform bildet die Wand der Verbindungs-Fluidführungseinheit zugleich eine äußere Wand für eine Kammer, welche Absorptionsmaterial aufnimmt, wobei diese Kammer zum CO2-Absorber gehört.

Das Merkmal, dass die Verbindungs-Fluidführungseinheit den CO2-Absorber umgibt, bewirkt in vielen Fällen, dass ein großflächiger und dadurch guter thermischer Kontakt zwischen dem CO2-Absorber und der Verbindungs-Fluidführungseinheit hergestellt ist. Dadurch kann der CO2-Absorber Wärme an die Verbindungs-Fluidführungseinheit abgeben. Bevorzugt wird diese Ausgestaltung kombiniert mit der Ausgestaltung, dass die Verbindungs-Fluidführungseinheit unmittelbar an die Umgebung angrenzt. Dadurch kann die Verbindungs-Fluidführungseinheit Wärme vom CO2-Absorber aufnehmen und an die Umgebung abgeben.

Dank der erfindungsgemäßen Absorptions-Anordnung hat das Gasgemisch einen niedrigeren Anteil an Kohlendioxid, wenn es aus der Absorptions-Anordnung austritt, verglichen mit dem Eintritt in die Absorptions-Anordnung, idealerweise überhaupt kein Kohlendioxid mehr. Dank des thermischen Kontakts mit der Umgebung und dank der Wasserfalle sind in vielen Fällen die Temperatur und der Feuchtegehalt aber beim Austritt aus der Absorptions-Anordnung nicht wesentlich höher oder sogar gleich oder niedriger als beim Eintritt.

In vielen Fällen steigt der Feuchtegehalt des Gasgemischs, während das Gasgemisch durch den CO2-Absorber fließt, insbesondere weil bei der exothermischen chemischen Reaktion im CO2-Absorber Wasser freigesetzt wird und / oder weil diese Reaktion zu einer gestiegenen Temperatur führt. Bevorzugt fließt das Gasgemisch senkrecht oder schräg nach oben durch die Abführ-Fluidführungseinheit oder wenigstens senkrecht oder schräg nach oben durch einen Abschnitt der Abführ-Fluidführungseinheit. Diese Ausgestaltung vergrößert weiter die Zuverlässigkeit, dass das Gasgemisch flussabwärts von der Wasserfalle keine Flüssigkeitstropfen mehr aufweist. Die Schwerkraft bewirkt in vielen Fällen, das Flüssigkeitstropfen die Wasserfalle nicht verlassen oder wenigstens wieder zurück zur Wasserfalle fließen.

Mindestens eines der Merkmale,
- dass das Gasgemisch senkrecht oder schräg nach unten durch die Verbindungs-Fluidführungseinheit zur Wasserfalle fließt und
- dass das Gasgemisch senkrecht oder schräg nach oben von der Wasserfalle weg durch die Abführ-Fluidführungseinheit fließt,
ist insbesondere dann von Vorteil, wenn das Gasgemisch sich im CO2-Absorber erwärmt und in einer nachfolgenden Fluidführungseinheit wieder abkühlt. In vielen Fällen kondensiert wenigstens ein Teil von Flüssigkeit im Gasgemisch an einer Wand dieser Fluidführungseinheit. Diese Flüssigkeit fließt dann senkrecht oder schräg nach unten in die Wasserfalle und wird dort gesammelt. Verhindert wird, dass diese Flüssigkeit die Absorptions-Anordnung verlässt.

In einer bevorzugten Ausgestaltung umfasst die Absorptions-Anordnung zusätzlich ein Zwischenstück. Dieses Zwischenstück ist sowohl mit der Quelle als auch mit der Senke verbunden oder lässt sich wenigstens zeitweise mit der Quelle und mit der Senke verbinden. Der CO2-Absorber und die Wasserfalle sind außerhalb des Zwischenstücks angeordnet und direkt oder indirekt mechanisch mit dem Zwischenstück verbunden. Bevorzugt ist das Zwischenstück als ein starres Bauteil, besonders bevorzugt mit mindestens einer Fluidführungseinheit im Inneren, ausgestaltet.

In einer Realisierungsform der Ausgestaltung mit den Zwischenstück lässt sich die mechanische Verbindung zwischen dem Zwischenstück und dem CO2-Absorber und / oder die mechanische Verbindung zwischen dem Zwischenstück und der Wasserfalle wieder lösen. Diese Realisierungsform erleichtert es, den CO2-Absorber bzw. die Wasserfalle auszutauschen.

Die Ausgestaltung mit dem Zwischenstück vermeidet die Notwendigkeit, den CO2-Absorber oder die Wasserfalle oder eine lose Fluidführungseinheit direkt mit der Quelle oder mit der Senke zu verbinden. Das Zwischenstück lässt sich als ein Adapter ausgestalten, so dass mehrere gleichartige CO2-Absorber und / oder mehrere gleichartige Wasserfallen sich mit mechanisch unterschiedlich ausgestalteten Quellen und / oder Senken verbinden lassen.

In einer Ausgestaltung umfasst die Absorptions-Anordnung ein Zwischenstück und ein weiteres Zwischenstück. Der CO2-Absorber und die Wasserfalle lassen sich wahlweise mit dem Zwischenstück oder mit dem weiteren Zwischenstück mechanisch verbinden. Die beiden Zwischenstücke können unterschiedliche Koppeleinheiten zur Verbindung mit einer Quelle und / oder mit einer Senke aufweisen. Die beiden Zwischenstücke fungieren also als unterschiedliche Adapter.

Die Ausgestaltung mit dem Zwischenstück und dem optionalen weiteren Zwischenstück ermöglicht es in vielen Fällen, einen bereits vorhandenen CO2-Absorber (genauer: einen bereits vorhandenen Typ eines CO2-Absorbers) und / oder eine bereits vorhandene Wasserfalle (genauer: einen Typ einer Wasserfalle) weiterzuverwenden. Der CO2-Absorber und / oder die Wasserfalle lassen sich mit dem Zwischenstück verbinden. Nicht erforderlich ist, eine bereits vorhandene Koppelstelle mit der Quelle oder eine bereits vorhandene Koppelstelle mit der Senke abzuändern. Diese Ausgestaltung erleichtert es, die erfindungsgemäße Absorptions-Anordnung in eine vorhandene Anlage zu integrieren.

Gemäß der Ausgestaltung mit dem Zwischenstück ist die Wasserfalle mechanisch mit dem Zwischenstück verbunden. In einer Realisierungsform befindet die Verbindungs-Fluidführungseinheit sich vollständig innerhalb des Zwischenstücks. In einer anderen Realisierungsform befindet sich die Verbindungs-Fluidführungseinheit - oder wenigstens ein Abschnitt der Verbindungs-Fluidführungseinheit - außerhalb des Zwischenstücks und ist mechanisch mit den Zwischenstück verbunden oder verbindbar, in einer Ausgestaltung lösbar verbunden oder verbindbar. Die Wasserfalle ist wiederum mit der Verbindungs-Fluidführungseinheit mechanisch verbunden. Möglich ist, dass sich ein Abschnitt der Verbindungs-Fluidführungseinheit im Zwischenstück und ein weiterer Abschnitt außerhalb des Zwischenstücks befinden.

In vielen Fällen führt die Ausgestaltung, dass die Verbindungs-Fluidführungseinheit oder wenigstens ein Abschnitt sich außerhalb des Zwischenstücks befindet, dazu, dass die Verbindungs-Fluidführungseinheit von einer Umgebung der Absorptions-Anordnung umgeben ist und dadurch Wärme an die Umgebung abgeben kann. Außerdem erleichtert diese Ausgestaltung ist in vielen Fällen, die Wasserfalle zu leeren und / oder durch eine neue Wasserfalle zu ersetzen.

In einer Realisierungsform befindet die Verbindungs-Fluidführungseinheit sich außerhalb des Zwischenstücks und ist flexibel ausgestaltet. In einer Realisierungsform befindet sich auch die Abführ-Fluidführungseinheit außerhalb des Zwischenstücks und ist flexibel ausgestaltet. Diese beiden Realisierungsformen verringern das Risiko, dass die Wasserfalle oder eine der Fluidführungseinheiten beschädigt wird, wenn die Absorptions-Anordnung mit einem anderen Gegenstand kollidiert.

In einer Realisierungsform der ersten Ausführungsform umfasst das Zwischenstück einen äußeren Adapter und einen inneren Adapter. Der äußere Adapter lässt sich mit der Quelle und mit der Senke verbinden. Der innere Adapter ist mit dem CO2-Absorber verbunden oder verbindbar. Die Wasserfalle ist mit dem äußeren Adapter oder mit dem inneren Adapter verbunden oder verbindbar.

Erfindungsgemäß fließt das Gasgemisch durch den CO2-Absorber, dann durch die obere Umlenk-Fluidführungseinheit und dann durch die Verbindungs-Fluidführungseinheit hindurch. In einer ersten Realisierungsform der Ausgestaltung mit den Zwischenstück befindet sich die obere Umlenk-Fluidführungseinheit - oder wenigstens ein Abschnitt der oberen Umlenk-Fluidführungseinheit - innerhalb des Zwischenstücks. Bei dieser ersten Realisierungsform schützt das Zwischenstück bis zu einem gewissen Grad die Umlenk-Fluidführungseinheit vor mechanischen Beschädigungen. In einer zweiten Realisierungsform befindet sich die obere Umlenk-Fluidführungseinheit - oder wenigstens ein Abschnitt der oberen Umlenk-Fluidführungseinheit - außerhalb des Zwischenstücks und ist mit dem Zwischenstück mechanisch verbunden. Die zweite Realisierungsform ermöglicht in vielen Fällen, dass die obere Umlenk-Fluidführungseinheit Wärme an die Umgebung abgibt.

In einer Ausgestaltung umfasst die Absorptions-Anordnung ein äußeres Gehäuse. Dieses äußere Gehäuse umgibt mindestens den CO2-Absorber, die Wasserfalle und alle erfindungsgemäß vorgesehenen Fluidführungseinheiten. Bevorzugt umgibt das äußere Gehäuse die gesamte Absorptions-Anordnung. Bevorzugt ist das äußere Gehäuse ein starres Gehäuse, welches für Fluid undurchlässig ist. Bevorzugt vermag das äußere Gehäuse einen großflächigen und dadurch guten thermischen Kontakt mit der Umgebung herzustellen, so dass Wärme, die bei der exothermischen chemischen Reaktion entsteht, an die Umgebung abgeführt wird. Die Ausgestaltung mit dem äußeren Gehäuse führt in vielen Fällen zu einer besonders kompakten und robusten Absorptions-Anordnung. Außerdem schützt das äußere Gehäuse die übrigen Bestandteile bis zu einem gewissen Grad vor mechanischer Beschädigung. In vielen Fällen ist es relativ einfach, die Absorptions-Anordnung zu greifen und auszutauschen.

In einer Realisierungsform der Ausgestaltung mit dem äußeren Gehäuse befindet die Verbindungs-Fluidführungseinheit - oder wenigstens ein Abschnitt der Verbindungs-Fluidführungseinheit - sich zwischen dem äußeren Gehäuse und dem CO2-Absorber, beispielsweise in Form einer Ummantelung. Möglich ist, dass das äußere Gehäuse zugleich eine äußere Wand der Verbindungs-Fluidführungseinheit bereitstellt. Möglich ist auch, dass eine Wand der Verbindungs-Fluidführungseinheit zugleich eine äußere Wand des CO2-Absorbers bereitstellt. Die gerade beschriebene Realisierungsform, nämlich dass die Verbindungs-Fluidführungseinheit sich zwischen dem äußeren Gehäuse und dem CO2-Absorber befindet, führt in vielen Fällen dazu, dass die Verbindungs-Fluidführungseinheit viel Wärme an die Umgebung abführen kann.

In einer Realisierungsform umfasst das äußere Gehäuse ein oberes Gehäuseteil und ein unteres Gehäuseteil. Die beiden Gehäuseteile sind mechanisch miteinander verbunden, und zwar in einer Ausgestaltung fest und in einer Ausgestaltung lösbar. Der obere Gehäuseteil umgibt wenigstens den CO2-Absorber, die beiden Umlenk-Fluidführungseinheiten und die Verbindungs-Fluidführungseinheit. Der untere Gehäuseteil bildet einen Boden der Wasserfalle. Die Bezeichnungen "oben" und "unten" beziehen sich wiederum auf eine Orientierung bei einem produktiven Einsatz der Absorptions-Anordnung.

Falls der untere Gehäuseteil lösbar mit dem oberen Gehäuseteil verbunden ist, so lässt die Wasserfalle sich sehr leicht vom oberen Gehäuseteil trennen, leeren und wieder verbinden, beispielsweise mit einem Schnappverschluss oder Rastverschluss oder Schraubverschluss. Beim produktiven Einsatz befindet der Boden der Wasserfälle sich unterhalb des oberen Gehäuseteils und damit auch unterhalb des CO2-Absorbers und der Fluidführungseinheiten. Dadurch fließt Flüssigkeit senkrecht oder schräg nach unten in die Wasserfalle.

Die Erfindung lässt sich dafür verwenden, um Kohlendioxid in folgender Anwendung aus einem Gasgemisch absorbieren: Das Gasgemisch fließt von einer patientenseitigen Koppeleinheit durch eine Fluidführungseinheit zu einem medizinischen Gerät, insbesondere zu einem Beatmungsgerät. Die patientenseitige Koppeleinheit ist im oder am oder auf dem Körper eines Patienten positioniert oder lässt sich dort positionieren. Die patientenseitige Koppeleinheit fungiert als Quelle, das medizinische Gerät als Senke. Die Fluidführungseinheit leitet also ausgeatmete Luft von der patientenseitigen Koppeleinheit zum medizinischen Gerät und hierbei durch die Absorptions-Anordnung hindurch. Diese ausgeatmete Luft fungiert als das Gasgemisch. Bekanntlich umfasst ausgeatmete Luft einen höheren Anteil an Kohlendioxid als die Umgebungsluft. Das medizinische Gerät erhält die ausgeatmete Luft, nachdem das ausgeatmete Gasgemisch durch die Absorptions-Anordnung geflossen ist und die erfindungsgemäße Absorptions-Anordnung Kohlendioxid aus der ausgeatmeten Luft herausgefiltert hat. In einer Ausgestaltung ist das medizinischen Gerät ein Beatmungsgerät. Das Beatmungsgerät vermag die empfangene Luft wieder zur patientenseitigen Koppeleinheit zu fördern. Das medizinische Gerät kann auch eine stationäre oder mobile Aufnahme für die ausgeatmete Luft sein.

Die Erfindung betrifft weiterhin ein Beatmungs-System zur künstlichen Beatmung eines Patienten. Dieses Beatmungs-System umfasst
- ein Beatmungsgerät,
- eine patientenseitige Koppeleinheit,
- eine Inspirations-Fluidverbindung,
- eine Exspirations-Fluidverbindung und
- eine erfindungsgemäße Absorptions-Anordnung.

Die patientenseitige Koppeleinheit ist im oder am Körper des Patienten positioniert oder lässt sich dort positionieren. Das Beatmungsgerät ist durch die beiden Fluidverbindungen mit der patientenseitigen Koppeleinheit verbunden. Die beiden Fluidverbindungen sind pneumatisch so voneinander isoliert, dass kein Fluid von der einen Fluidverbindung direkt in die andere Fluidverbindung fließen kann.

Das Beatmungsgerät vermag ein Gasgemisch durch die Inspirations-Fluidverbindung hindurch zur patientenseitigen Koppeleinheit zu fördern.

Ein Gasgemisch kann beim Einsatz des Beatmungs-Systems von der patientenseitigen Koppeleinheit durch die Exspirations-Fluidverbindung hindurch zum Beatmungsgerät fließen. Die patientenseitige Koppeleinheit fungiert als eine Quelle für ein Gasgemisch, in diesem Falle als Quelle für ausgeatmete Luft. Das Beatmungsgerät fungiert als eine Senke für das Gasgemisch.

Beim Vorgang, dass das Gasgemisch (ausgeatmete Luft) von der patientenseitigen Koppeleinheit (Quelle) zu dem Beatmungsgerät (Senke) fließt, fließt das Gasgemisch durch die erfindungsgemäße Absorptions-Anordnung hindurch. Die Absorptions-Anordnung filtert Kohlendioxid aus diesem Gasgemisch heraus.

Im Folgenden wird die Erfindung anhand eines Ausführungsbeispiels beschrieben. Hierbei zeigt
- Figur 1: schematisch die Verwendung der erfindungsgemäßen Absorptions-Anordnung in einem Beatmungskreislauf;
- Figur 2: in einer Querschnittsdarstellung und aus einer Blickrichtung von oben einen CO2-Absorber ohne Wasserfalle;
- Figur 3: das koppelseitige Zwischenstück der ersten Ausführungsform;
- Figur 4: die erste Ausführungsform in zwei Seitenansichten aus zwei verschiedenen Blickrichtungen;
- Figur 5: die erste Ausführungsform in einer Seitenansicht und einer Querschnittsdarstellung aus derselben Blickrichtung;
- Figur 6: schematisch die zweite Ausführungsform in einer Querschnittsdarstellung;
- Figur 7: die zweite Ausführungsform in einer perspektivischen Querschnittsdarstellung und in einer perspektivischen Ansicht von außen.

Im Ausführungsbeispiel wird die Erfindung in einem Beatmungskreislauf verwendet. In diesem Beatmungskreislauf zirkuliert ein Gasgemisch zwischen einem Beatmungsgerät und der Lunge L eines Patienten P. Das Beatmungsgerät hält den Fluss des Gasgemisches im Beatmungskreislauf aufrecht. Ein Inspirations-Gasgemisch umfassend Sauerstoff und mindestens ein Narkosemittel fließt vom Beatmungsgerät durch eine Inspirations-Fluidverbindung zu dem Patienten und wird vom Patienten eingeatmet. Ein Exspirations-Gasgemisch umfassend Kohlendioxid (CO2) wird vom Patienten ausgeatmet und fließt durch eine Exspirations-Fluidverbindung zurück zum Beatmungsgerät. Weil ein weitgehend fluiddichter Beatmungskreislauf hergestellt ist, tritt kein Narkosemittel in die Umgebung aus.

Um das Exspirations-Gasgemisch wieder dem Patienten P zum Einatmen zuführen zu können, muss dem Exspirations-Gasgemisch das ausgeatmete Kohlendioxid entzogen werden. Zu diesem Zweck wird das Exspirations-Gasgemisch durch einen CO2-Absorber geleitet. Ein Absorptionsmaterial in diesem CO2-Absorber, bevorzugt umfassend Atemkalk, bindet das Kohlendioxid an sich und entzieht es dadurch dem Exspirations-Gasgemisch, welches durch den CO2-Absorber geleitet wird.

Die chemische Reaktion im CO2-Absorber ist im Ausführungsbeispiel exothermisch, d. h. das Exspirations-Gasgemisch erwärmt sich. Außerdem steigt der Feuchtegehalt im Exspirations-Gasgemisch. Daher wird Feuchtigkeit im Exspirations-Gasgemisch zur Kondensation gebracht und sammelt sich in einem Wasserbehälter, der zu einer sogenannten Wasserfalle gehört. Die Erfindung betrifft eine Absorptions-Anordnung mit dem CO2-Absorber und der Wasserfalle.

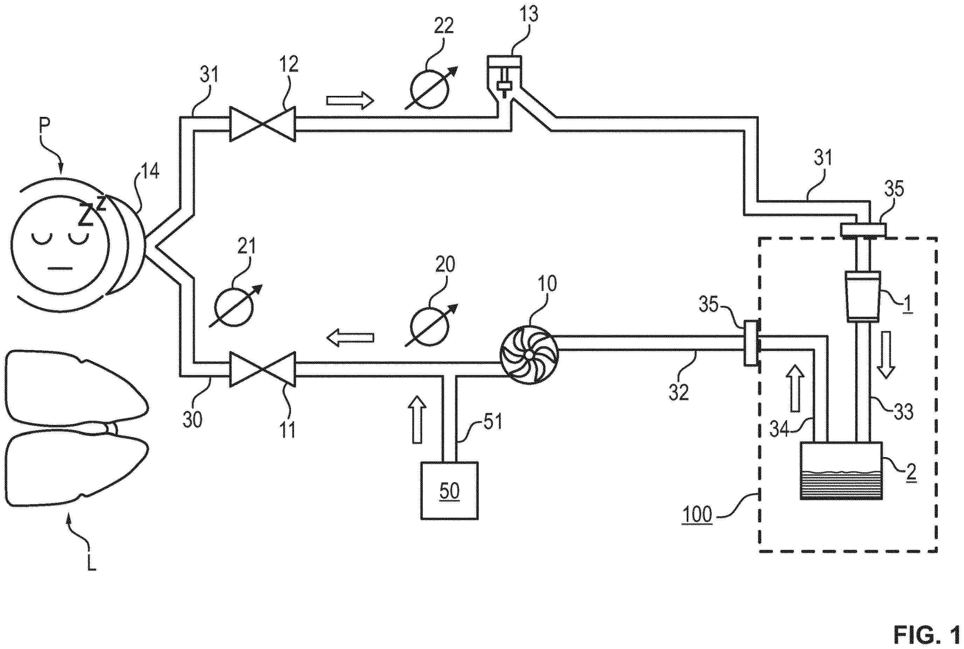

Figur 1 zeigt schematisch einige Bestandteile dieses Beatmungskreislaufs. Gezeigt werden:
- eine erfindungsgemäße Absorptions-Anordnung 100, welche einen CO2-Absorber 1 und eine Wasserfalle 2 umfasst,
- eine patientenseitige Koppeleinheit 14, welche in oder am Körper des Patienten P positioniert ist, beispielsweise eine Atemmaske oder ein Tubus oder ein Katheter,
- ein Gebläse 10 eines nicht gezeigten Beatmungsgeräts, wobei das Gebläse 10 den Gasfluss im Beatmungskreislauf aufrecht erhält,
- eine Inspirations-Leitung 30, die vom Gebläse 10 zur patientenseitigen Koppeleinheit 14 führt,
- einen Narkosemitteldosierer 50, der ein Narkotisierungs-Gasgemisch umfassend mindestens ein Narkosemittel erzeugt,
- eine Narkosemittel-Zuführleitung 51, die vom Narkosemitteldosierer 50 in die Inspirations-Leitung 30 führt und das Narkotisierungs-Gasgemisch mit dem Narkosemittel in die Inspirations-Leitung 30 einspeist,
- eine Exspirations-Leitung mit einem ersten Abschnitt 31, der von der patientenseitigen Koppeleinheit 14 zu der Absorptions-Anordnung 100 führt, und einem zweiten Abschnitt 32, der von der Absorptions-Anordnung 100 zum Gebläse 10 führt, und
- eine leitungsseitige Koppeleinheit 35 an den beiden Abschnitten 31 und 32, durch die die Absorptions-Anordnung 100 sich lösbar mit beiden Abschnitten 31 und 32 der Exspirations-Leitung verbinden lässt.

Möglich ist auch, dass eine andere Fluidförderereinheit den Gasfluss im Beatmungskreislauf aufrechterhält, beispielsweise eine Kolben-Zylinder-Einheit oder ein Handbeatmungsbeutel.

Die Absorptions-Anordnung 100 umfasst
- den CO2-Absorber 1,
- die Wasserfalle 2,
- eine Zwischen-Verbindungsleitung 33, die vom CO2-Absorber 1 zur Wasserfalle 2 führt und als Verbindungs-Fluidführungseinheit fungiert, und
- eine Abführ-Verbindungsleitung 34, die von der Wasserfalle 2 zum zweiten Abschnitt 32 führt.

Das Exspirations-Gasgemisch fließt vom ersten Abschnitt 31 zur Absorptions-Anordnung 100 und dort durch den CO2-Absorber 1 hindurch, durch die Zwischen-Verbindungsleitung 33, die Wasserfalle 2 und die Abführ-Verbindungsleitung 34 hindurch und dann in den zweiten Abschnitt 32.

Der CO2-Absorber 1 entzieht dem Gasgemisch, welches durch den CO2-Absorber 1 fließt, Kohlendioxid. Das von Kohlendioxid gereinigte Exspirations-Gasgemisch fließt durch die Zwischen-Verbindungsleitung 33 zur Wasserfalle 2, wo Feuchtigkeit im Exspirations-Gasgemisch kondensiert und in einem Behälter der Wasserfalle 2 aufgefangen wird. Dadurch wird dem Exspirations-Gasgemisch Feuchtigkeit entzogen

Ein Lungendruck-Ventil (PEEP-Saver) 13 im ersten Abschnitt 31 gewährleistet, dass ein endexspiratorischer Druck (Positive End-Expiratory Pressure) in der Lunge des Patienten P eingehalten wird, und leitet einen Überdruck im ersten Abschnitt 31 in die Umgebung ab. Ein Druck-Sensor 20 misst ein Maß für den Druck in der Inspirations-Leitung 30, ein Druck-Sensor 22 für den Druck in der Exspirations-Leitung 31. Ein Volumenfluss-Sensor 21 misst ein Maß für den Volumenfluss in der Inspirations-Leitung 30. Ein nicht gezeigtes signalverarbeitendes Steuergerät (control unit) erhält und verarbeitet Messwerte von den Sensoren 20, 21 und 22 und steuert abhängig von den Messwerten ein Inspirations-Ventil 11 und ein Exspirations-Ventil 12 an. Das Steuergerät steuert das Inspirations-Ventil 11 mit dem Regelungsziel an, dass der tatsächliche Verlauf des Volumenflusses durch die oder auch der tatsächliche Verlauf des Drucks in der Inspirations-Leitung 30 einem vorgegebenen zeitlichen Verlauf folgt. Entsprechend steuert das Steuergerät das Exspirations-Ventil 12 mit dem Regelungsziel an, dass der tatsächliche Verlauf des Volumenflusses durch die oder auch der tatsächliche Verlauf des Drucks in dem ersten Abschnitt 31 der Exspirations-Leitung einem vorgegebenen zeitlichen Verlauf folgt.

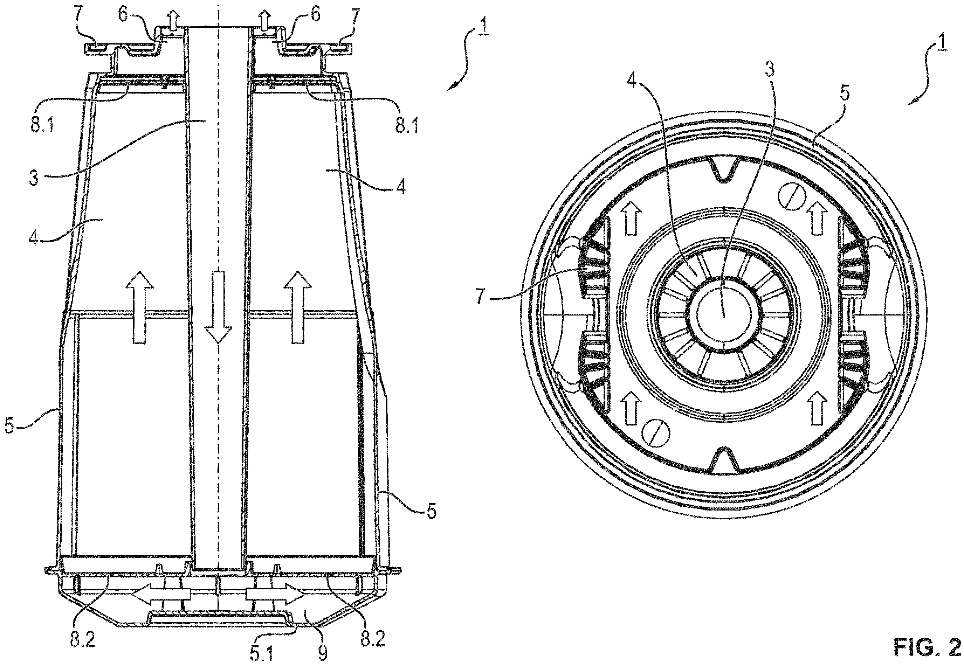

Figur 2 zeigt einen beispielhaften CO2-Absorber 1, und zwar links in einer Schnittdarstellung und rechts in einer Ansicht von oben. Dieser CO2-Absorber 1 kann ein Bestandteil einer Ausführungsform der erfindungsgemäßen Absorptions-Anordnung 100 sein. Der CO2-Absorber 1 gemäß Figur 2 hat die Form einer Kartusche und umfasst
- ein bevorzugt starres Gehäuse 5 mit einem Boden 5.1,
- ein Absorptionsmaterial 4, welches CO2 zu absorbieren vermag und beispielsweise Atemkalk umfasst,
- eine Zuführ-Röhre 3,
- einen Abführ-Hohlraum 6 oberhalb des Absorptionsmaterials 4,
- ein oberes Sieb 8.1 umfassend einen Staubschutzvlies, wobei das obere Sieb 8.1 zwischen dem Abführ-Hohlraum 6 und dem Absorptionsmaterial 4 angeordnet ist,
- einen unteren Umlenk-Hohlraum 9 unterhalb des Absorptionsmaterials 4 und oberhalb des Bodens 5.1,
- ein unteres Sieb 8.2 zwischen dem Absorptionsmaterial 4 und dem Umlenk-Hohlraum 9 und
- einen Adapter 7, der den Abführ-Hohlraum 6 umgibt.

Das Gehäuse 5 stellt einen Behälter für das Absorptionsmaterial 4 bereit. Dieser Behälter ist nach unten durch das untere Sieb 8.2 und nach oben durch das obere Sieb 8.1 begrenzt. Die Zuführ-Röhre 3 ist mittig durch das Absorptionsmaterial 4 hindurch geführt. Der Abführ-Hohlraum 6 steht in einer Fluidverbindung mit dem Absorptionsmaterial 4, wobei diese Fluidverbindung durch das obere Sieb 8.1 hindurch führt.

Mithilfe des Adapters 7 lässt der CO2-Absorber 1 sich lösbar an der korrespondierenden leitungsseitigen Koppeleinheit 35 der Exspirations-Leitung 31, 32 befestigen, beispielsweise mithilfe einer Schnappverbindung oder Rastverbindung oder Schraubverbindung. Wenn der CO2-Adapter 1 verbunden ist, so steht die Zuführ-Röhre 3 in einer Fluidverbindung mit dem ersten Abschnitt 31 der Exspirations-Leitung. Der Abführ-Hohlraum 6 steht über die Zwischen-Verbindungsleitung 33 in einer Fluidverbindung mit der Wasserfalle 2 und damit indirekt in einer Fluidverbindung mit dem zweiten Abschnitt 32 der Exspirations-Leitung.

Falls der CO2-Absorber 1 an der leitungsseitigen Koppeleinheit 35 befestigt ist, so ist die Zuführ-Röhre 3 vertikal angeordnet, und das Gehäuse 5 befindet sich unterhalb des Adapters 7. Die Bezeichnungen "oben" und "unten" beziehen sich auf eine Orientierung des CO2-Absorbers 1, die dann auftritt, wenn dieser produktiv eingesetzt wird und an der leitungsseitigen Koppeleinheit 35 befestigt ist. Das untere Sieb 8.2 ist für Gas durchlässig, nicht aber für Absorptionsmaterial 4, und verhindert, dass Absorptionsmaterial 4 in den Umlenk-Hohlraum 9 gelangt. Der Abführ-Hohlraum 6 befindet sich oberhalb des Absorptionsmaterials 4. Das Exspirations-Gasgemisch fließt durch die Zuführ-Röhre 3 nach unten, wird am Boden 5.1 des Gehäuses 5 umgelenkt und fließt durch den Umlenk-Hohlraum 9 und das untere Sieb 8.2 und dann durch das Absorptionsmaterial 4 nach oben. Das Absorptionsmaterial 4 entzieht dem durchfließenden Exspirations-Gasgemisch Kohlendioxid. Das von Kohlendioxid gereinigte Exspirations-Gasgemisch gelangt in den Abführ-Hohlraum 6 und dann in die Verbindungsleitung 33. Die Pfeile in Figur 2 deuten an, wie das Gasgemisch durch den CO2-Absorber 1 fließt. Das Exspirations-Gasgemisch wird durch die Konstruktion der Absorptions-Anordnung 100 dazu gezwungen, durch den gesamten CO2-Absorber 1 hindurchzufließen. Solange der CO2-Absorber 1 überhaupt noch absorptionsfähiges Material umfasst, wird auch Kohlendioxid absorbiert. Dies könnte nicht der Fall sein, wenn das Exspirations-Gasgemisch zuvor aus dem CO2-Absorber entweichen könnte.

Anmerkung: In internen Versuchen haben die Erfinder empirisch festgestellt, dass das CO2-Absorptionsmaterial 4 mehr Kohlendioxid aufnehmen kann, wenn das Exspirations-Gasgemisch von senkrecht oder schräg unten nach oben durch das Absorptionsmaterial 4 fließt, fließt, als wenn es von oben nach unten oder waagrecht durchfließt.

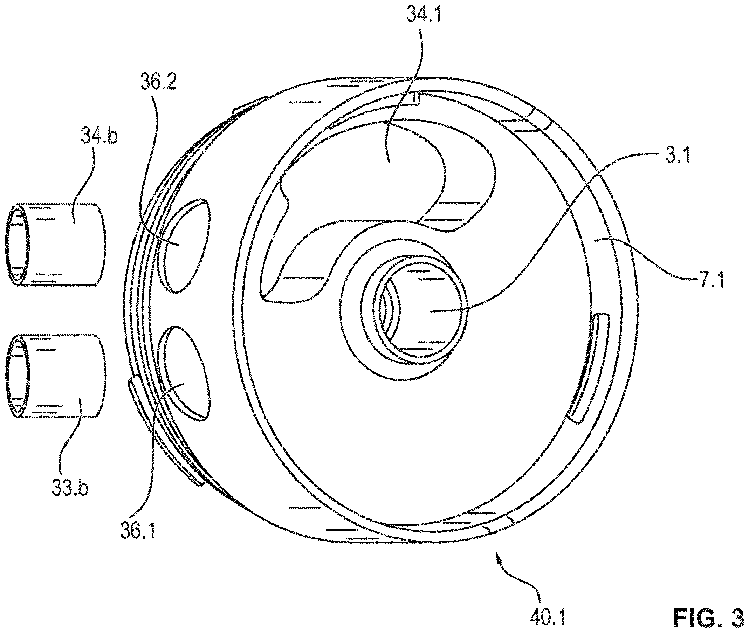

Figur 3 bis Figur 5 zeigen eine erste Ausführungsform der erfindungsgemäßen Absorptions-Anordnung 100. Die Absorptions-Anordnung 100 gemäß der ersten Ausführungsform umfasst
- den CO2-Absorber 1 von Figur 2,
- die Wasserfalle 2 mit einem Reservoir für Wasser oberhalb eines Bodens 2.1,
- die Zwischen-Verbindungsleitung 33,
- die Abführ-Verbindungsleitung 34,
- ein koppelseitiges Zwischenstück 40.1 und
- ein absorberseitiges Zwischenstück 40.2.

Das koppelseitige Zwischenstück 40.1 von Figur 3 umfasst einen Adapter 7.1. Dieser Adapter 7.1 lässt sich genauso mit der leitungsseitigen Koppeleinheit 35 verbinden wie der Adapter 7 des CO2-Absorbers 1 von Figur 2, 2, bevorzugt lösbar verbinden. Dank eines Adapters 7.2 lässt sich das absorberseitige Zwischenstück 40.2 mit dem koppelseitigen Zwischenstück 40.1 verbinden, bevorzugt lösbar verbinden. Der CO2-Absorber 1 ist mit dem absorberseitigen Zwischenstück 40.2 verbunden, bevorzugt lösbar verbunden.

In das koppelseitige Zwischenstück 40.1 sind eine koppelseitige Fortsetzung 3.1 der Zuführ-Röhre 3 und eine koppelseitige Fortsetzung 34.1 der Abführ-Verbindungsleitung 34 eingelassen. In das absorberseitige Zwischenstück 40.2 sind eine Fortsetzung 3.2 der Zuführ-Röhre 3 und eine Fortsetzung 6.2 des Abführ-Hohlraums 6 eingelassen.

Die beiden Verbindungsleitungen 33 und 34 umfassen jeweils einen Faltenschlauch 33.a, 34.a sowie eine starre gebogene Röhre 33.b, 34.b. Die beiden starren Röhren 33.b, 34.b umfassen jeweils ein Segment in Form eines Viertelkreises und zwei angrenzende gerade Segmente und lassen sich in zwei korrespondierende Aufnahmen 36.1, 36.2 im koppelseitige Zwischenstück 40.1 einsetzen oder sind fest in diese Aufnahmen 36.1, 36.2 eingesetzt. Weil die beiden Faltenschläuche 33.a, 34.a flexibel sind, ist die Gefahr geringer, dass eine Verbindungsleitung 33, 34 oder die Wasserfalle 2 abbricht, wenn die Absorptions-Anordnung 100 mit einem starren Gegenstand kollidiert. Bevorzugt ist die Wasserfalle 2 lösbar mit den beiden Faltenschläuchen 33.a, 34.a verbunden, so dass sich die Wasserfalle 2 lösen und entleeren lässt.

Die Zuführ-Röhre 3 und die Fortsetzungen 3.1, 3.2 fungieren bei der ersten Ausführungsform als die Zuführ-Fluidführungseinheit. Die Zwischen-Verbindungsleitung 33 und der Faltenschlauch 33.a fungieren als die Verbindungs-Fluidführungseinheit, welche den CO2-Absorber 1 mit der Wasserfalle 2 verbindet. Der Faltenschlauch 34.a, die starre Röhre 34.b und die Fortsetzung 34.1 gehören zu der Abführ-Fluidführungseinheit.

Wie bereits dargelegt, wird bei dem Vorgang, dass das Absorptionsmaterial 4 Kohlendioxid aufnimmt, Wärme freigesetzt. Dadurch wird das Exspirations-Gasgemisch beim Durchfließen erwärmt. Die Absorptions-Anordnung 100 vermag an mehreren Stellen Wärme in die Umgebung abzugeben.

Das Absorptionsmaterial 4 umgibt die Zuführ-Röhre 3. Das erwärmte Absorptionsmaterial 4 kann daher Wärme an die Zuführ-Röhre 3 abgeben. Das dadurch erwärmte Gasgemisch wird im unteren Umlenk-Hohlraum 9 umgelenkt, und der Boden des unteren Umlenk-Hohlraums 9 kann Wärme an die Umgebung abgeben.

Die beiden Verbindungsleitungen 33 und 34 stehen in einem thermischen Kontakt mit der Umgebung. Die Umgebung umgibt vollständig die beiden Verbindungsleitungen 33 und 34. Nachdem das Gasgemisch durch das Absorptionsmaterial 4 geflossen ist, hat es sich erwärmt. Durch den thermischen Kontakt mit der Umgebung kühlt das Gasgemisch sich ab, und Wassertropfen kondensieren an den inneren Wänden der Verbindungsleitungen 33 und 34. Diese Wassertropfen laufen nach unten in die Wasserfalle 2.

Das Gasgemisch fließt auf folgendem Wege vom ersten Abschnitt 31 durch die Absorptions-Anordnung 100 gemäß der ersten Ausführungsform zum zweiten Abschnitt 32:
- nach unten durch die Fortsetzung 3.1,
- nach unten durch die Fortsetzung 3.2,
- nach unten durch die Zuführ-Röhre 3,
- durch den Umlenk-Hohlraum 9, wo das Gasgemisch umgelenkt wird,
- nach oben durch das Absorptionsmaterial 4, wo Kohlendioxid absorbiert wird,
- nach oben durch den Abführ-Hohlraum 6,
- nach oben durch die Fortsetzung 6.2, wo das Gasgemisch in eine waagerechte Richtung umgelenkt wird,
- durch die Zuführ-Verbindungsleitung 33, und zwar erst entlang einer Kurve durch die Röhre 33.b, so dass das Gasgemisch nach unten umgelenkt wird, und dann nach unten durch den Faltenschlauch 33.a,
- durch die Wasserfalle 2, wo sich Kondenswasser sammelt,
- durch die Abführ-Verbindungsleitung 34, und zwar erst nach oben durch den Faltenschlauch 34.a und dann durch die Röhre 34.b entlang einer Kurve, und dann
- durch die Fortsetzung 34.1.

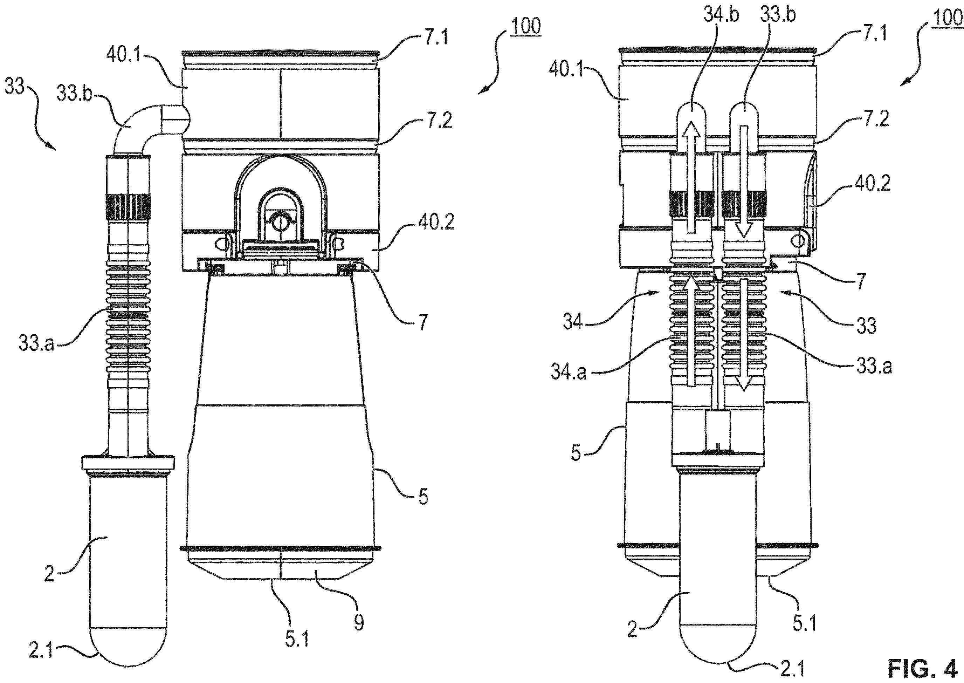

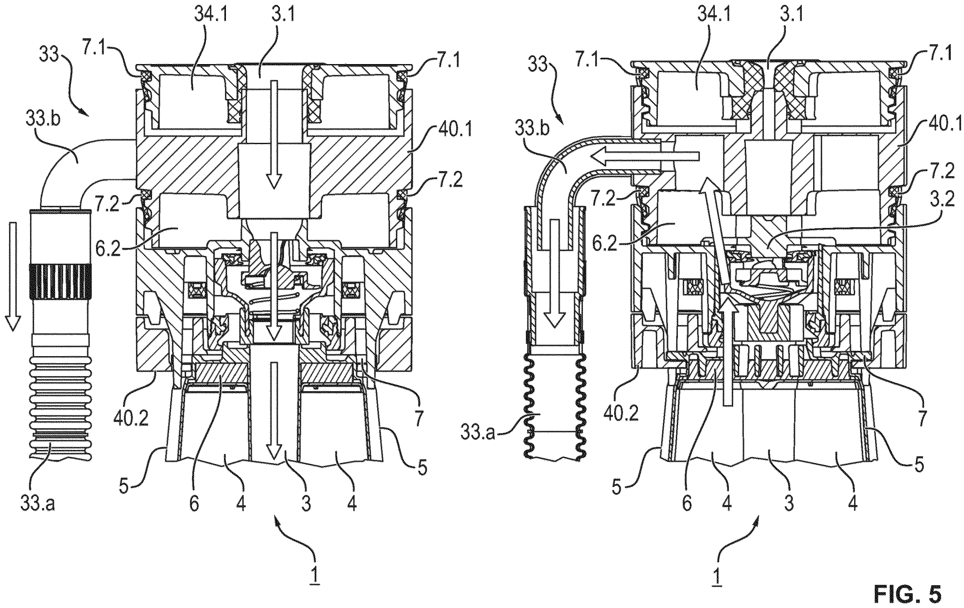

Dieser Weg ist in Figur 4 und Figur 5 durch Pfeile angedeutet. In der ersten Ausführungsform fungieren die Fortsetzung 6.2 und die Röhre 33.b als die obere Umlenk-Fluidführungseinheit, der Umlenk-Hohlraum 9 als die untere Umlenk-Fluidführungseinheit. In einer Realisierungsform lenken beide Umlenk-Fluidführungseinheiten 9, 6.2, 33.b das Gasgemisch um jeweils 180 Grad um.

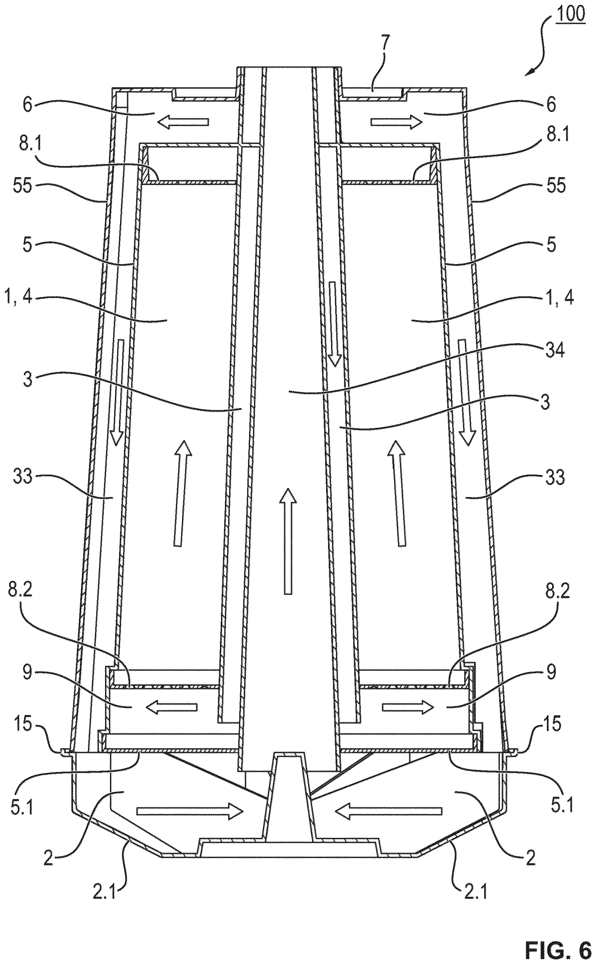

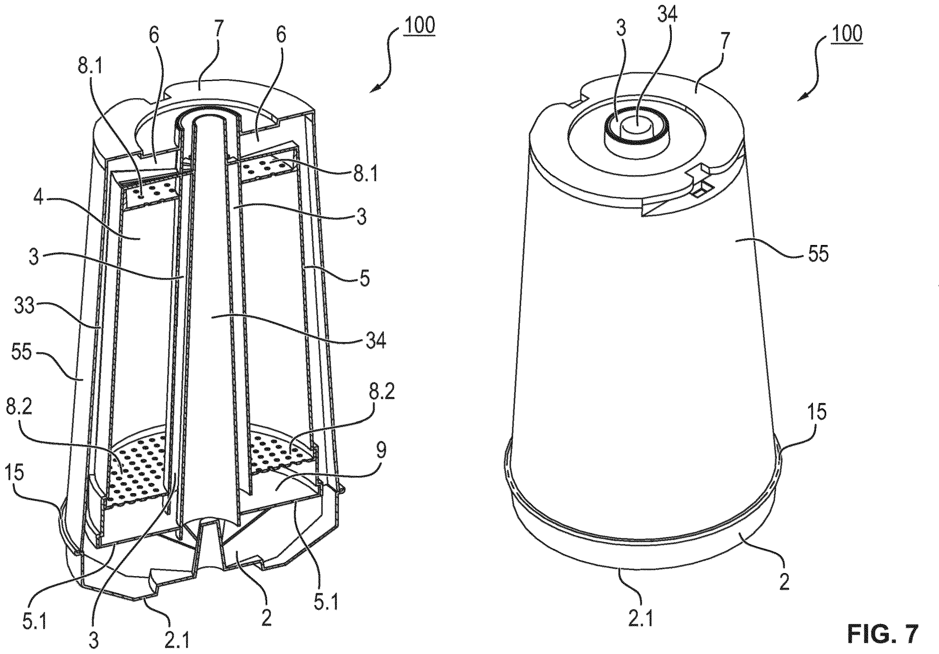

Figur 6 und Figur 7 zeigen eine zweite Ausführungsform der erfindungsgemäßen Absorptions-Anordnung 100. Die gesamte Absorptions-Anordnung 100 ist bei dieser zweiten Ausführungsform als eine Kartusche realisiert. Dadurch wird eine besonders kompakte und mechanisch stabile und robuste Realisierung gewährleistet.

Figur 6 zeigt das Prinzip in einer schematischen Querschnittsdarstellung. Einander entsprechenden Bestandteile haben das gleiche Bezugszeichen wie in Figur 3 bis Figur 5. Die Absorptions-Anordnung 100 hat bei der zweiten Ausführungsform ein äußeres Gehäuse, welches sowohl den CO2-Absorber 1 und die Wasserfalle 2 als auch die Zuführ-Röhre 3 und die Verbindungsleitungen 33, 34 aufnimmt und vollständig umgibt und bevorzugt starr ist. Dieses äußere Gehäuse umfasst einen sich nach unten verbreiternden oberen Teil 55 sowie als unteren Teil den Boden 2.1 der Wasserfalle 2. Die Wasserfalle 2 befindet sich unterhalb des CO2-Absorbers 1, und der Boden 2.1 ist mit dem oberen Gehäuseteil 55 mechanisch verbunden. In einer Ausgestaltung ist der Boden 2.1 durch eine Rastverbindung 15 lösbar mit dem oberen Gehäuseteil 55 verbunden, in einer anderen Ausgestaltung fest verbunden. Auch die Zwischen-Verbindungsleitung 33 und die Abführ-Verbindungsleitung 34 befinden sich im Inneren des oberen Gehäuseteils 55. Die Zwischenstücke 40.1 und 40.2 sind bei der zweiten Ausführungsform nicht erforderlich.

In der gezeigten Ausführungsform tritt zwischen dem Gehäuse 5 des CO2-Absorbers 1 und dem oberen Gehäuseteil 55 ein röhrenförmiger Spalt auf, der als die Zwischen-Verbindungsleitung 33 fungiert. Dank dieser Ausgestaltung steht die Zwischen-Verbindungsleitung 33 in einer großen Fläche, nämlich über mindestens die Hälfte der Mantelfläche des oberen Gehäuseteils 55, in thermischem Kontakt mit der Umgebung. Dadurch wird ein Gasgemisch abgekühlt, während es durch die röhrenförmige Zwischen-Verbindungsleitung 33 nach unten fließt.

In der gezeigten Realisierung ist die Abführ-Verbindungsleitung 34 koaxial durch das Innere der Zuführ-Röhre 3 geführt. Möglich ist auch, dass die Zuführ-Röhre 3 koaxial durch das Innere der Abführ-Verbindungsleitung 34 geführt ist.

Oben am oberen Gehäuseteil 55 ist ein Adapter 7 angebracht. Dieser Adapter 7 kann so ausgestaltet sein wie der Adapter 7 des CO2-Absorbers 1 von Figur 2. Dank dieses Adapters 7 lässt sich die Absorptions-Anordnung 100 lösbar mit der leitungsseitige Koppeleinheit 35 verbinden.

Das Gasgemisch fließt auf folgendem Wege vom ersten Abschnitt 31 durch die Absorptions-Anordnung 100 gemäß der zweiten Ausführungsform zum zweiten Abschnitt 32:
- nach unten durch die Zuführ-Röhre 3 im Inneren des CO2-Absorbers 1,
- durch den unteren Umlenk-Hohlraum 9, wo das Gasgemisch umgelenkt wird,
- nach oben durch das Absorptionsmaterial 4, wo Kohlendioxid absorbiert wird,
- nach oben durch den Abführ-Hohlraum 6, wo das Gasgemisch erneut umgelenkt wird,
- nach unten durch die Zwischen-Verbindungsleitung 33, wobei das Gasgemisch abgekühlt wird und Wasser an der Innenwand des oberen Gehäuseteils 55 und in manchen Fällen an der Außenwand des Gehäuses 5 kondensiert,
- in die Wasserfalle 2, wo das Gasgemisch erneut umgelenkt wird, und
- nach oben durch die Abführ-Verbindungsleitung 34.

Die Zuführ-Röhre 3 fungiert als die Zuführ-Fluidführungseinheit der zweiten Ausführungsform. Der untere Umlenk-Hohlraum 9 fungiert als die untere Umlenk-Fluidführungseinheit, der Abführ-Hohlraum 6 als die obere Umlenk-Fluidführungseinheit. Die Zwischen-Verbindungsleitung 33 fungiert als die Verbindungs-Fluidführungseinheit, die als die Abführ-Fluidführungseinheit.

Figur 7 veranschaulicht die zweite Ausführungsform links in einer perspektivischen Querschnittsdarstellung und rechts in einer perspektivischen Darstellung von außen. Das oberen Gehäuseteil 55 hat die Form eines umgedrehten Topf. Der Boden 2.1 der Wasserfalle 2 ist über eine lösbare oder feste Verbindung 15 mit dem oberen Gehäuseteil 55 verbunden. Im Falle einer lösbaren Verbindung lässt sich die Wasserfalle 2 sich daher vom oberen Gehäuseteil 55 lösen, entleeren und wieder verbinden.

Bei der zweiten Ausführungsform wird insbesondere an folgenden Stellen Wärme an die Umgebung abgegeben:
- Die Zuführ-Röhre 3 befindet sich zwischen dem Absorptionsmaterial 4 und der Abführ-Verbindungsleitung 34. Dadurch kann das Absorptionsmaterial viel Wärme an die Zuführ-Röhre 3 abgeben, und die Zuführ-Röhre 3 kann Wärme an die Abführ-Verbindungsleitung 34 abgeben.
- Die Zwischen-Verbindungsleitung 33 befindet sich zwischen dem Absorptionsmaterial 4 und dem oberen Gehäuseteil 55. Dadurch kann die Zwischen-Verbindungsleitung 33 Wärme durch den oberen Gehäuseteil 55 hindurch an die Umgebung abgeben.

### Bezugszeichenliste

| | |
|---|---|
| 1 | CO2-Absorber, umfasst die Zuführ-Röhre 3, das CO2-Absorptionsmaterial 4, das Gehäuse 5, den Abführ-Hohlraum 6, den Adapter 7, die beiden Siebe 8.1 und 8.2 und den Umlenk-Hohlraum 9 |
| 2 | Wasserfalle, wird oberhalb des Bodens 2.1 gebildet |
| 2.1 | Boden der Wasserfalle 2 |
| 3 | mittig angeordnete vertikale Zuführ-Röhre des CO2-Absorbers 1, steht in einer Fluidverbindung mit dem ersten Abschnitt 31 |
| 3.1 | Fortsetzung der Zuführ-Röhre 3 in dem koppelseitigen Zwischenstück 40.1 |
| 4 | CO2-Absorptionsmaterial im Gehäuse 5 |
| 5 | Gehäuse des CO2-Absorbers 1, nimmt das Absorptionsmaterial 4 und die Hohlräume 6, 9 auf, hat den Boden 5.1 |
| 5.1 | Boden des Gehäuses 5 |
| 6 | Abführ-Hohlraum, steht in einer Fluidverbindung mit der Zwischen-Verbindungsleitung 33 |
| 6.2 | Fortsetzung des Abführ-Hohlraums 6 im absorberseitigen Zwischenstück 40.2 |
| 7 | Adapter des CO2-Absorbers 1, lässt sich lösbar mit der leitungsseitigen Koppeleinheit 35 verbinden |
| 7.1 | Adapter, mit dem sich das koppelseitige Zwischenstück 40.1 mit der leitungsseitigen Koppeleinheit 35 verbinden lässt |
| 7.2 | Adapter, mit dem sich das absorberseitige Zwischenstück 40.2 mit dem koppelseitigen Zwischenstück 40.1 verbinden lässt |
| 8.1 | oberes Sieb zwischen dem Absorptionsmaterial 4 und dem Abführ-Hohlraum |
| 8.2 | unteres Sieb zwischen dem Absorptionsmaterial 4 und dem Umlenk-Hohlraum 9 |
| 9 | Umlenk-Hohlraum zwischen dem unteren Sieb 8.2 und dem Boden 5.1 des CO2-Absorbers 1 |
| 10 | Gebläse des Beatmungsgeräts, hält einen Gasfluss im Beatmungskreislauf aufrecht |
| 11 | Inspirations-Ventil, bewirkt einen geregelten Volumenfluss durch die Inspirations-Leitung 30 |
| 12 | Exspirations-Ventil, bewirkt einen geregelten Volumenfluss durch die Exspirations-Leitung 31, 32 |
| 13 | Lungendruck-Ventil (PEEP-Saver) im ersten Abschnitt 31, gewährleistet das Einhalten eines endexspiratorischen Lungendrucks |
| 14 | patientenseitige Koppeleinheit, mit den Leitungen 30 und 31 verbunden |
| 15 | lösbare Verbindung zwischen dem Boden 2.1 der Wasserfalle 2 und dem Gehäuseteil 55 |
| 20 | Druck-Sensor, misst den Druck in der Inspirations-Leitung 30 |
| 21 | Volumenfluss-Sensor, misst den Volumenfluss in der Inspirations-Leitung 30 |
| 22 | Druck-Sensor, misst den Druck in der Exspirations-Leitung 31 |
| 30 | Inspirations-Leitung, führt vom Gebläse 10 zur patientenseitigen Koppeleinheit 14 |
| 31 | erster Abschnitt der Exspirations-Leitung, führt von der patientenseitigen Koppeleinheit 14 zu der Absorptions-Anordnung 100 |
| 32 | zweiter Abschnitt der Exspirations-Leitung, führt von der Absorptions-Anordnung 100 zum Gebläse 10 |
| 33 | Zwischen-Verbindungsleitung, führt vom CO2-Absorber 1 zur Wasserfalle 2 |
| 33. a | Faltenschlauch der Zuführ-Verbindungsleitung 33 |
| 34. b | starre Röhre der Zuführ-Verbindungsleitung 33 |
| 34 | Abführ-Verbindungsleitung, führt von der Wasserfalle 2 zum zweiten Abschnitt 32 |
| 34.1 | Fortsetzung der Abführ-Verbindungsleitung 34 in dem koppelseitigen Zwischenstück 40.1 |
| 34. a | Faltenschlauch der Abführ-Verbindungsleitung 34 |
| 34. b | starre Röhre der Abführ-Verbindungsleitung 34 |
| 35 | leitungsseitige Koppeleinheit zwischen der Absorptions-Anordnung 100 einerseits und den beiden Abschnitten 31 und 32 der Exspirations-Leitung andererseits |
| 36.1 | Aufnahme im koppelseitigen Zwischenstück 40.1 für die Röhre 33.b der Zuführ-Verbindungsleitung 33 |
| 36.2 | Aufnahme im koppelseitigen Zwischenstück 40.1 für die Röhre 34.b der Abführ-Verbindungsleitung 34 |
| 40.1 | koppelseitiges Zwischenstück, ist zwischen dem CO2-Adapter 1 und der leitungsseitigen Koppeleinheit 35 angeordnet |
| 40.2 | absorberseitiges Zwischenstück, ist zwischen dem koppelseitigen Zwischenstück 40.1 und dem CO2-Adapter 1 angeordnet |
| 50 | Narkosemitteldosierer, erzeugt ein Gasgemisch umfassend ein Narkosemittel |
| 51 | Narkosemittel-Zuführleitung, mündet in die Inspirations-Leitung 30 |
| 55 | oberes Gehäuseteil des äußeres Gehäuses der Absorptions-Anordnung 100 gemäß der zweiten Ausführungsform, umgibt den CO2-Absorber 1 und die Verbindungsleitungen 33, 34, trägt die Wasserfalle 2 |
| 100 | Anordnung mit dem Absorber 1 und der Wasserfalle 2 |
| L | Lunge des Patienten P |
| P | Patient, hat die Lunge L, ist mit der patientenseitigen Koppeleinheit 14 verbunden |

## Patentansprüche

1. Absorptions-Anordnung (100) umfassend
- einen CO2-Absorber (1),
- eine Wasserfalle (2),
- eine Zuführ-Fluidführungseinheit (3, 3.1, 3.2),
- eine untere Umlenk-Fluidführungseinheit (9),
- eine obere Umlenk-Fluidführungseinheit (6, 6.2, 33.b),
- eine Abführ-Fluidführungseinheit (34, 34.1) und
- eine Verbindungs-Fluidführungseinheit (33, 33.a), welche den CO2-Absorber (1) mit der Wasserfalle (2) verbindet,
wobei die Zuführ-Fluidführungseinheit (3, 3.1, 3.2) mit einer Quelle (31) für ein Gasgemisch 3.2) wenigstens zeitweise verbunden oder verbindbar ist,
wobei die Abführ-Fluidführungseinheit (34, 34.1) mit einer Senke (32) für ein Gasgemisch wenigstens zeitweise verbunden oder verbindbar ist,
wobei der CO2-Absorber (1) dazu ausgestaltet ist, Kohlendioxid zu absorbieren und dadurch aus einem Gasgemisch, welches durch den CO2-Absorber (1) strömt, Kohlendioxid herauszufiltern,
wobei die Wasserfalle (2) dazu ausgestaltet ist, eine Feuchtigkeit aufzunehmen,
wobei die Absorptions-Anordnung (100) so ausgestaltet ist, dass ein Gasgemisch aus der Quelle (31)
- zuerst durch die Zuführ-Fluidführungseinheit (3, 3.1, 3.2),
- dann durch die untere Umlenk-Fluidführungseinheit (9),
- dann durch den CO2-Absorber (1),
- dann durch die obere Umlenk-Fluidführungseinheit (6, 6.2, 33.b),
- dann durch die Verbindungs-Fluidführungseinheit (33, 33.a),
- dann durch die Wasserfalle (2) und
- dann durch die Abführ-Fluidführungseinheit (34, 34.1) hindurch zur Senke (32) fließt,
wobei die Absorptions-Anordnung (100) so ausgestaltet ist, dass bei einem Einsatz der Absorptions-Anordnung (100) das Gasgemisch
- von der unteren Umlenk-Fluidführungseinheit (9) senkrecht oder schräg nach oben umgelenkt wird,
- senkrecht oder schräg nach oben durch den CO2-Absorber (1) (1) hindurch fließt,
- von der oberen Umlenk-Fluidführungseinheit (6, 6.2, 33.b) senkrecht oder schräg nach unten umgelenkt wird und
- senkrecht oder schräg nach unten durch die Verbindungs-Fluidführungseinheit (33, 33.a) zur Wasserfalle (2) fließt.

2. Absorptions-Anordnung (100) nach Anspruch 1,
**dadurch gekennzeichnet, dass** sich bei einem Einsatz der Absorptions-Anordnung (100)
- die untere Umlenk-Fluidführungseinheit (9) unterhalb des CO2-Absorbers (1) befindet und
- der CO2-Absorber (1) unterhalb der oberen Umlenk-Fluidführungseinheit (6, 6.2, 33.b) befindet.

3. Absorptions-Anordnung (100) nach einem der vorhergehenden Ansprüche,
**dadurch gekennzeichnet, dass**
die Verbindungs-Fluidführungseinheit (33, 33.a) unmittelbar an eine Umgebung der Absorptions-Anordnung (100) angrenzt und
bevorzugt vollständig von der Umgebung umgeben ist.

4. Absorptions-Anordnung (100) nach einem der vorhergehenden Ansprüche,
**dadurch gekennzeichnet, dass**
der CO2-Absorber (1) die Zuführ-Fluidführungseinheit (3, 3.1, 3.2) oder wenigstens einen Abschnitt (3) der Zuführ-Fluidführungseinheit (3, 3.1, 3.2) nach Art einer Ummantelung umgibt.

5. Absorptions-Anordnung (100) nach einem der vorhergehenden Ansprüche,
**dadurch gekennzeichnet, dass**
die Verbindungs-Fluidführungseinheit (33, 33.a) oder wenigstens ein Abschnitt (33) der Verbindungs-Fluidführungseinheit (33, 33.a) den CO2-Absorber (1) nach Art einer Ummantelung umgibt.

6. Absorptions-Anordnung (100) nach einem der vorhergehenden Ansprüche,
**dadurch gekennzeichnet, dass**
das Absorptions-Anordnung (100) zusätzlich ein Zwischenstück (40.1, 40.2) umfasst,
wobei das Zwischenstück (40.1, 40.2) wenigstens zeitweise sowohl mit der Quelle (31) als auch mit der Senke (32) verbunden oder verbindbar ist und wobei der CO2-Absorber (1) und die Wasserfalle (2) mit dem Zwischenstück (40.1, 40.2) mechanisch verbunden oder verbindbar sind,
bevorzugt lösbar mechanisch verbunden.

7. Absorptions-Anordnung (100) nach Anspruch 6,
**dadurch gekennzeichnet, dass**
die Verbindungs-Fluidführungseinheit (33, 33.a) mechanisch mit den Zwischenstück (40.1, 40.2) verbunden ist und
die Wasserfalle (2) mechanisch mit der Verbindungs-Fluidführungseinheit (33, 33.a) und über die Verbindungs-Fluidführungseinheit (33, 33.a) mit dem Zwischenstück (40.1, 40.2) verbunden ist.

8. Absorptions-Anordnung (100) nach Anspruch 6 oder Anspruch 7,
**dadurch gekennzeichnet, dass**
ein Abschnitt (6, 6.2) der oberen Umlenk-Fluidführungseinheit (6, 6.2, 33.b) sich im Zwischenstück (40.1, 40.2) befindet und / oder
ein Abschnitt (33.b) der oberen Umlenk-Fluidführungseinheit (6, 6.2, 33.b) mit dem Zwischenstück (40.1, 40.2) verbunden ist.

9. Absorptions-Anordnung (100) nach einem der Ansprüche 6 bis 8,
**dadurch gekennzeichnet, dass**
das Absorptions-Anordnung (100) ein weiteres Zwischenstück umfasst,
wobei das weitere Zwischenstück (40.1, 40.2)
- mit der Quelle (31) oder einer anderen Quelle für ein Gasgemisch und
- mit der Senke (32) oder eine anderen Senke für ein Gasgemisch verbunden oder verbindbar ist und
wobei der CO2-Absorber (1) und die Wasserfalle (2) wahlweise mit dem Zwischenstück (40.1, 40.2) oder mit dem weiteren Zwischenstück mechanisch verbunden oder verbindbar sind.

10. Absorptions-Anordnung (100) nach einem der vorhergehenden Ansprüche,
**dadurch gekennzeichnet, dass**
die Absorptions-Anordnung (100) ein äußeres Gehäuse (55, 2.1) umfasst,
wobei das äußere Gehäuse (55, 2.1)
- den CO2-Absorber (1),
- die Wasserfalle (2),
- die Zuführ-Fluidführungseinheit (3, 3.1, 3.2),
- die untere Umlenk-Fluidführungseinheit (9),
- die obere Umlenk-Fluidführungseinheit (6, 6.2, 33.b),
- die Abführ-Fluidführungseinheit (34, 34.1) und
- die Verbindungs-Fluidführungseinheit (33, 33.a
umgibt.

11. Absorptions-Anordnung (100) nach Anspruch 10,
**dadurch gekennzeichnet, dass**
die Verbindungs-Fluidführungseinheit (33, 33.a) sich zwischen einem Segment (55) des äußeren Gehäuses (55, 2.1) und dem CO2-Absorber (1) befindet.

12. Absorptions-Anordnung (100) nach Anspruch 10 oder Anspruch 11,
**dadurch gekennzeichnet, dass**
das äußere Gehäuse (55, 2.1)
- ein oberes Gehäuseteil (55) und
- ein unteres Gehäuseteil (2.1), das mechanisch mit dem oberen Gehäuseteil (55) verbunden ist,
umfasst,
wobei die Absorptions-Anordnung (100) so ausgestaltet ist, dass bei einem Einsatz der Absorptions-Anordnung (100) der obere Gehäuseteil (55) sich oberhalb des unteren Gehäuseteils (2.1) befindet,
wobei das obere Gehäuseteil (55) den CO2-Absorber (1), die beiden Umlenk-Fluidführungseinheiten (6, 9) und die Verbindungs-Fluidführungseinheit (33, 33.a) umgibt und
wobei das untere Gehäuseteil (2.1) einen Boden der Wasserfalle (2) bildet.

13. Verwendung einer Absorptions-Anordnung (100) nach einem der vorhergehenden Ansprüche
zur Absorption von Kohlendioxid aus einem Gasgemisch,
wobei das Gasgemisch in einer Exspirations-Fluidverbindung (31, 32) von einer patientenseitige Koppeleinheit (14),
die im oder am oder auf dem Körper eines Patienten (P) positioniert oder positionierbar ist und als die Quelle für das Gasgemisch fungiert,
zu einem medizinischen Gerät, insbesondere zu einem Beatmungsgerät (10), fließt,
wobei das medizinische Gerät als die Senke für das Gasgemisch fungiert.

14. Beatmungs-System zur künstlichen Beatmung eines Patienten (P),
wobei das Beatmungs-System
- ein Beatmungsgerät (10),
- eine patientenseitige Koppeleinheit (14), die im oder am Körper des Patienten (P) positioniert oder positionierbar ist,
- eine Inspirations-Fluidverbindung (30),
- eine Exspirations-Fluidverbindung (31, 32) und
- eine Absorptions-Anordnung (100) nach einem der Ansprüche 1 bis 12 umfasst,
wobei das Beatmungsgerät (10) mit der patientenseitigen Koppeleinheit (14) durch die beiden Fluidverbindungen (30, 31, 32) miteinander verbunden ist,
wobei das Beatmungsgerät (10) dazu ausgestaltet ist, ein Gasgemisch durch die Inspirations-Fluidverbindung (30) hindurch zur patientenseitigen Koppeleinheit (14) zu fördern,
wobei das Beatmungs-System so ausgestaltet ist, dass
- ein Gasgemisch von der Exspirations-Fluidverbindung (31, 32) durch die Exspirations-Fluidverbindung (31, 32) hindurch zum Beatmungsgerät (10) fließt und
- beim Fließen durch die Exspirations-Fluidverbindung (31, 32) durch die Absorptions-Anordnung (100) hindurchfließt.

15. Verfahren zum Herausfiltern von Kohlendioxid aus einem Gasgemisch
unter Verwendung einer Absorptions-Anordnung (100), die
- einen CO2-Absorber (1),
- eine Wasserfalle (2),
- eine Zuführ-Fluidführungseinheit (3, 3.1, 3.2),
- eine untere Umlenk-Fluidführungseinheit (9),
- eine obere Umlenk-Fluidführungseinheit (6, 6.2, 33.b),
- eine Abführ-Fluidführungseinheit (34, 34.1) und
- eine Verbindungs-Fluidführungseinheit (33, 33.a), welche den CO2-Absorber (1) mit der Wasserfalle (2) verbindet,
umfasst,
wobei das Verfahren die Schritte umfasst, dass das Gasgemisch
- von einer Quelle (31) zur Zuführ-Fluidführungseinheit (3, 3.1, 3.2) geleitet wird,
- durch die Zuführ-Fluidführungseinheit (3, 3.1, 3.2) hindurch zur unteren Umlenk-Fluidführungseinheit (9) geleitet wird,
- von der unteren Umlenk-Fluidführungseinheit (9) senkrecht oder schräg nach oben umgelenkt wird,
- senkrecht oder schräg nach oben durch den CO2-Absorber (1) hindurchfließt,
- von der oberen Umlenk-Fluidführungseinheit (6, 6.2, 33.b) senkrecht oder schräg nach unten umgelenkt wird,
- senkrecht oder schräg nach unten durch die Verbindungs-Fluidführungseinheit (33, 33.a) 33.a) hindurch zur Wasserfalle (2) fließt,
- durch die Wasserfalle (2) hindurchfließt,
- durch die Abführ-Fluidführungseinheit (34, 34.1) hindurchfließt und
- von der Abführ-Fluidführungseinheit (34, 34.1) zu einer Senke (32) geleitet wird,
wobei das Verfahren den weiteren Schritt umfasst, dass
der CO2-Absorber (1) Kohlendioxid absorbiert, während das Gasgemisch durch den CO2-Absorber (1) fließt, und dadurch Kohlendioxid aus dem Gasgemisch herausfiltert.

## Claims

1. Absorption assembly (100) comprising
- a CO2 absorber (1),
- a water trap (2),
- a supply fluid guide unit (3, 3.1, 3.2),
- a lower deflection fluid guide unit (9),
- an upper deflection fluid guide unit (6, 6.2, 33.b),
- a removal fluid guide unit (34, 34.1), and
- a connecting fluid guide unit (33, 33.a) which connects the CO2 absorber (1) to the water trap (2),
wherein the supply fluid guide unit (3, 3.1, 3.2) is at least temporarily connected or connectable to a source (31) of a gas mixture (3.2),
wherein the removal fluid guide unit (34, 34.1) is at least temporarily connected or connectable to a sink (32) for a gas mixture,
wherein the CO2 absorber (1) is designed to absorb carbon dioxide and thereby filter out carbon dioxide from a gas mixture flowing through the CO2 absorber (1),
wherein the water trap (2) is designed to absorb moisture,
wherein the absorption assembly (100) is designed such that a gas mixture flows from the source (31)
- first through the supply fluid guide unit (3, 3.1, 3.2),
- then through the lower deflection fluid guide unit (9),
- then through the CO2 absorber (1),
- then through the upper deflection fluid guide unit (6, 6.2, 33.b),
- then through the connecting fluid guide unit (33, 33.a),
- then through the water trap (2), and
- then through the removal fluid guide unit (34, 34.1)
to the sink (32),
wherein the absorption assembly (100) is designed such that, when the absorption assembly (100) is used, the gas mixture
- is deflected vertically or diagonally upward by the lower deflection fluid guide unit (9),
- flows vertically or diagonally upward through the CO2 absorber (1) (1),
- is deflected vertically or diagonally downward by the upper deflection fluid guide unit (6, 6.2, 33.b), and
- flows vertically or diagonally downward through the connecting fluid guide unit (33, 33.a) to the water trap (2).

2. Absorption assembly (100) according to claim 1,
**characterized in that,** when the absorption assembly (100) is used,
- the lower deflection fluid guide unit (9) is located below the CO2 absorber (1), and
- the CO2 absorber (1) is located below the upper deflection fluid guide unit (6, 6.2, 33.b).

3. Absorption assembly (100) according to either of the preceding claims,
**characterized in that**
the connecting fluid guide unit (33, 33.a) is directly adjacent to an environment of the absorption assembly (100) and
preferably completely surrounded by the environment.

4. Absorption assembly (100) according to any of the preceding claims,
**characterized in that**
the CO2 absorber (1) surrounds the supply fluid guide unit (3, 3.1, 3.2) or at least a portion (3) of the supply fluid guide unit (3, 3.1, 3.2) like a casing.

5. Absorption assembly (100) according to any of the preceding claims,
**characterized in that**
the connecting fluid guide unit (33, 33.a) or at least a portion (33) of the connecting fluid guide unit (33, 33.a) surrounds the CO2 absorber (1) like a casing.

6. Absorption assembly (100) according to any of the preceding claims,
**characterized in that**
the absorption assembly (100) additionally comprises a connector (40.1, 40.2),
the connector (40.1, 40.2) being at least temporarily connected or connectable both to the source (31) and to the sink (32), and
the CO2 absorber (1) and the water trap (2) being mechanically connected or connectable to the connector (40.1, 40.2),
preferably detachably mechanically connected.

7. Absorption assembly (100) according to claim 6,
**characterized in that**
the connecting fluid guide unit (33, 33.a) is mechanically connected to the connector (40.1, 40.2), and
the water trap (2) is mechanically connected to the connecting fluid guide unit (33, 33.a) and, via the connecting fluid guide unit (33, 33.a), to the connector (40.1, 40.2).

8. Absorption assembly (100) according to either claim 6 or claim 7,
**characterized in that**
a portion (6, 6.2) of the upper deflection fluid guide unit (6, 6.2, 33.b) is located in the connector (40.1, 40.2), and/or
a portion (33.b) of the upper deflection fluid guide unit (6, 6.2, 33.b) is connected to the connector (40.1, 40.2).

9. Absorption assembly (100) according to any of claims 6 to 8,
**characterized in that**
the absorption assembly (100) comprises a further connector,
the further connector (40.1, 40.2) being connected or connectable
- to the source (31) or another source of a gas mixture, and
- to the sink (32) or another sink for a gas mixture,
and
the CO2 absorber (1) and the water trap (2) being optionally mechanically connected or connectable to the connector (40.1, 40.2) or to the further connector.

10. Absorption assembly (100) according to any of the preceding claims,
**characterized in that**
the absorption assembly (100) comprises an outer housing (55, 2.1),
the outer housing (55, 2.1) surrounding
- the CO2 absorber (1),
- the water trap (2),
- the supply fluid guide unit (3, 3.1, 3.2),
- the lower deflection fluid guide unit (9),
- the upper deflection fluid guide unit (6, 6.2, 33.b),
- the removal fluid guide unit (34, 34.1), and
- the connecting fluid guide unit (33, 33.a).

11. Absorption assembly (100) according to claim 10,
**characterized in that**
the connecting fluid guide unit (33, 33.a) is located between a segment (55) of the outer housing (55, 2.1) and the CO2 absorber (1).

12. Absorption assembly (100) according to either claim 10 or claim 11,
**characterized in that**
the outer housing (55, 2.1) comprises
- an upper housing part (55), and
- a lower housing part (2.1) which is mechanically connected to the upper housing part (55),
the absorption assembly (100) being designed such that, when the absorption assembly (100) is used, the upper housing part (55) is located above the lower housing part (2.1),
the upper housing part (55) surrounding the CO2 absorber (1), the two deflection fluid guide units (6, 9) and the connecting fluid guide unit (33, 33.a), and
the lower housing part (2.1) forming a bottom of the water trap (2).

13. Use of an absorption assembly (100) according to any of the preceding claims
for absorption of carbon dioxide from a gas mixture,
wherein the gas mixture, in an expiration fluid connection (31, 32), flows from a patient-side coupling unit (14),
which is positioned or positionable in or on the body of a patient (P) and acts as the source of the gas mixture,
to a medical device, in particular to a ventilation device (10),
wherein the medical device acts as the sink for the gas mixture.

14. Ventilation system for artificial ventilation of a patient (P),
wherein the ventilation system comprises
- a ventilation device (10),
- a patient-side coupling unit (14) which is positioned or positionable in or on the body of the patient (P),
- an inspiration fluid connection (30),
- an expiration fluid connection (31, 32), and
- an absorption assembly (100) according to any of claims 1 to 12,
wherein the ventilation device (10) is connected to the patient-side coupling unit (14) by the two fluid connections (30, 31, 32),
wherein the ventilation device (10) is designed to convey a gas mixture through the inspiration fluid connection (30) to the patient-side coupling unit (14),
wherein the ventilation system is designed such that
- a gas mixture flows from the expiration fluid connection (31, 32) through the expiration fluid connection (31, 32) to the ventilation device (10), and
- while flowing through the expiration fluid connection (31, 32), flows through the absorption assembly (100).

15. Method for filtering out carbon dioxide from a gas mixture
using an absorption assembly (100) which comprises
- a CO2 absorber (1),
- a water trap (2),
- a supply fluid guide unit (3, 3.1, 3.2),
- a lower deflection fluid guide unit (9),
- an upper deflection fluid guide unit (6, 6.2, 33.b),
- a removal fluid guide unit (34, 34.1), and
- a connecting fluid guide unit (33, 33.a) which connects the CO2 absorber (1) to the water trap (2),
wherein the method comprises the steps of the gas mixture
- being conveyed from a source (31) to the supply fluid guide unit (3, 3.1, 3.2),
- being conveyed through the supply fluid guide unit (3, 3.1, 3.2) to the lower deflection fluid guide unit (9),
- being deflected vertically or diagonally upward by the lower deflection fluid guide unit (9),
- flowing vertically or diagonally upward through the CO2 absorber (1),
- being deflected vertically or diagonally downward by the upper deflection fluid guide unit (6, 6.2, 33.b),
- flowing vertically or diagonally downward through the connecting fluid guide unit (33, 33.a) 33.a) to the water trap (2),
- flowing through the water trap (2),
- flowing through the removal fluid guide unit (34, 34.1), and
- being conveyed from the removal fluid guide unit (34, 34.1) to a sink (32),
wherein the method comprises the further step of
the CO2 absorber (1) absorbing carbon dioxide while the gas mixture flows through the CO2 absorber (1), and thereby filtering carbon dioxide out of the gas mixture.

## Revendications

1. Agencement d'absorption (100) comprenant
- un absorbeur de CO2 (1),
- un piège à eau (2),
- une unité de guidage de fluide d'alimentation (3, 3.1, 3.2),
- une unité inférieure de guidage de fluide de déviation (9),
- une unité supérieure de guidage de fluide de déviation (6, 6.2, 33.b),
- une unité de guidage de fluide d'évacuation (34, 34.1) et
- une unité de guidage de fluide de liaison (33, 33.a) qui relie l'absorbeur de CO2 (1) au piège à eau (2),
dans lequel l'unité de guidage de fluide d'alimentation (3, 3.1, 3.2) est reliée ou peut être reliée au moins temporairement à une source (31) pour un mélange de gaz (3.2),
dans lequel l'unité de guidage de fluide d'évacuation (34, 34.1) est reliée ou peut être reliée au moins temporairement à un collecteur (32) pour un mélange de gaz,
dans lequel l'absorbeur de CO2 (1) est conçu pour absorber du dioxyde de carbone et ainsi filtrer le dioxyde de carbone hors d'un mélange de gaz qui s'écoule à travers l'absorbeur de CO2 (1),
dans lequel le piège à eau (2) est conçu pour recevoir de l'humidité,
dans lequel l'agencement d'absorption (100) est conçu de sorte qu'un mélange de gaz provenant de la source (31) s'écoule
- d'abord à travers l'unité de guidage de fluide d'alimentation (3, 3.1, 3.2),
- puis à travers l'unité inférieure de guidage de fluide de déviation (9),
- puis à travers l'absorbeur de CO2 (1),
- puis à travers l'unité supérieure de guidage de fluide de déviation (6, 6.2, 33.b),
- puis à travers l'unité de guidage de fluide de liaison (33, 33.a),
- puis à travers le piège à eau (2) et
- puis à travers l'unité de guidage de fluide d'évacuation (34, 34.1)
jusqu'au collecteur (32),
dans lequel l'agencement d'absorption (100) est conçu de sorte que, lors d'une utilisation de l'agencement d'absorption (100), le mélange de gaz
- est dévié verticalement ou obliquement vers le haut par l'unité inférieure de guidage de fluide de déviation (9),
- s'écoule verticalement ou obliquement vers le haut à travers l'absorbeur de CO2 (1) (1),
- est dévié verticalement ou obliquement vers le bas par l'unité supérieure de guidage de fluide de déviation (6, 6.2, 33.b), et
- s'écoule verticalement ou obliquement vers le bas à travers l'unité de guidage de fluide de liaison (33, 33.a) jusqu'au piège à eau (2).

2. Agencement d'absorption (100) selon la revendication 1,
**caractérisé en ce que,** lors d'une utilisation de l'agencement d'absorption (100),
- l'unité inférieure de guidage de fluide de déviation (9) se situe en dessous de l'absorbeur de CO2 (1) et
- l'absorbeur de CO2 (1) se situe en dessous de l'unité supérieure de guidage de fluide de déviation (6, 6.2, 33.b).

3. Agencement d'absorption (100) selon l'une des revendications précédentes,
**caractérisé en ce que**
l'unité de guidage de fluide de liaison (33, 33.a) est directement adjacente à un environnement de l'agencement d'absorption (100) et
est de préférence complètement entourée par l'environnement.

4. Agencement d'absorption (100) selon l'une des revendications précédentes,
**caractérisé en ce que**
l'absorbeur de CO2 (1) entoure l'unité de guidage de fluide d'alimentation (3, 3.1, 3.2) ou au moins une section (3) de l'unité de guidage de fluide d'alimentation (3, 3.1, 3.2) à la manière d'une gaine.

5. Agencement d'absorption (100) selon l'une des revendications précédentes,
**caractérisé en ce que**
l'unité de guidage de fluide de liaison (33, 33.a) ou au moins une section (33) de l'unité de guidage de fluide de liaison (33, 33.a) entoure l'absorbeur de CO2 (1) à la manière d'une gaine.

6. Agencement d'absorption (100) selon l'une des revendications précédentes,
**caractérisé en ce que**
l'agencement d'absorption (100) comprend en outre une pièce intermédiaire (40.1, 40.2),
dans lequel la pièce intermédiaire (40.1, 40.2) est reliée ou peut être reliée au moins temporairement aussi bien à la source (31) qu'au collecteur (32) et
dans lequel l'absorbeur de CO2 (1) et le piège à eau (2) sont reliés ou peuvent être reliés mécaniquement à la pièce intermédiaire (40.1, 40.2),
de préférence reliés mécaniquement de manière amovible.

7. Agencement d'absorption (100) selon la revendication 6,
**caractérisé en ce que**
l'unité de guidage de fluide de liaison (33, 33.a) est reliée mécaniquement à la pièce intermédiaire (40.1, 40.2) et
le piège à eau (2) est relié mécaniquement à l'unité de guidage de fluide de liaison (33, 33.a) et, par l'intermédiaire de l'unité de guidage de fluide de liaison (33, 33.a), à la pièce intermédiaire (40.1, 40.2).

8. Agencement d'absorption (100) selon la revendication 6 ou la revendication 7,
**caractérisé en ce que**
une section (6, 6.2) de l'unité supérieure de guidage de fluide de déviation (6, 6.2, 33.b) se situe dans la pièce intermédiaire (40.1, 40.2) et/ou
une section (33.b) de l'unité supérieure de guidage de fluide de déviation (6, 6.2, 33.b) est reliée à la pièce intermédiaire (40.1, 40.2).

9. Agencement d'absorption (100) selon l'une des revendications 6 à 8,
**caractérisé en ce que**
l'agencement d'absorption (100) comprend une pièce intermédiaire supplémentaire,
dans lequel la pièce intermédiaire (40.1, 40.2) supplémentaire est reliée ou peut être reliée
- à la source (31) ou à une autre source pour un mélange de gaz et
- au collecteur (32) ou à un autre collecteur pour un mélange de gaz
et
dans lequel l'absorbeur de CO2 (1) et le piège à eau (2) sont reliés ou peuvent être reliés mécaniquement, au choix, à la pièce intermédiaire (40.1, 40.2) ou à la pièce intermédiaire supplémentaire.

10. Agencement d'absorption (100) selon l'une des revendications précédentes,
**caractérisé en ce que**
l'agencement d'absorption (100) comprend un boîtier extérieur (55, 2.1),
dans lequel le boîtier extérieur (55, 2.1) entoure
- l'absorbeur de CO2 (1),
- le piège à eau (2),
- l'unité de guidage de fluide d'alimentation (3, 3.1, 3.2),
- l'unité inférieure de guidage de fluide de déviation (9),
- l'unité supérieure de guidage de fluide de déviation (6, 6.2, 33.b),
- l'unité de guidage de fluide d'évacuation (34, 34.1) et
- l'unité de guidage de fluide de liaison (33, 33.a).

11. Agencement d'absorption (100) selon la revendication 10,
**caractérisé en ce que**
l'unité de guidage de fluide de liaison (33, 33.a) se situe entre un segment (55) du boîtier extérieur (55, 2.1) et l'absorbeur de CO2 (1).

12. Agencement d'absorption (100) selon la revendication 10 ou la revendication 11,
**caractérisé en ce que**
le boîtier extérieur (55, 2.1) comprend
- une partie supérieure de boîtier (55) et
- une partie inférieure de boîtier (2.1) qui est reliée mécaniquement à la partie supérieure de boîtier (55),
dans lequel l'agencement d'absorption (100) est conçu de sorte que, lors d'une utilisation de l'agencement d'absorption (100), la partie supérieure de boîtier (55) se situe au-dessus de la partie inférieure de boîtier (2.1),
dans lequel la partie supérieure de boîtier (55) entoure l'absorbeur de CO2 (1), les deux unités de guidage de fluide de déviation (6, 9) et l'unité de guidage de fluide de liaison (33, 33.a) et
dans lequel la partie inférieure de boîtier (2.1) forme un fond du piège à eau (2).

13. Utilisation d'un agencement d'absorption (100) selon l'une des revendications précédentes
pour l'absorption de dioxyde de carbone d'un mélange de gaz,
dans laquelle le mélange de gaz s'écoule, dans un moyen de liaison fluidique d'expiration (31, 32), depuis une unité d'accouplement (14) côté patient,
laquelle est positionnée ou peut être positionnée dans, sur le corps d'un patient (P) ou au niveau de celui-ci et agit comme la source pour le mélange de gaz,
jusqu'à un appareil médical, en particulier jusqu'à un appareil d'assistance respiratoire (10),
dans laquelle l'appareil médical agit comme collecteur pour le mélange de gaz.

14. Système d'assistance respiratoire pour la respiration artificielle d'un patient (P),
dans lequel le système d'assistance respiratoire comprend
- un appareil d'assistance respiratoire (10),
- une unité d'accouplement (14) côté patient, laquelle est positionnée ou peut être positionnée dans le corps du patient (P) ou au niveau de celui-ci,
- un moyen de liaison fluidique d'inspiration (30),
- un moyen de liaison fluidique d'expiration (31, 32) et
- un agencement d'absorption (100) selon l'une des revendications 1 à 12,
dans lequel l'appareil d'assistance respiratoire (10) est relié l'un à l'autre à l'unité d'accouplement (14) côté patient au moyen des deux moyens de liaison fluidique (30, 31, 32),
dans lequel l'appareil d'assistance respiratoire (10) est conçu pour transporter un mélange de gaz à travers le moyen de liaison fluidique d'inspiration (30) jusqu'à l'unité d'accouplement (14) côté patient,
dans lequel le système d'assistance respiratoire est conçu de sorte que
- un mélange de gaz s'écoule depuis le moyen de liaison fluidique d'expiration (31, 32), à travers le moyen de liaison fluidique d'expiration (31, 32), jusqu'à l'appareil d'assistance respiratoire (10) et
- lors de l'écoulement à travers le moyen de liaison fluidique d'expiration (31, 32), s'écoule à travers l'agencement d'absorption (100).

15. Procédé permettant de filtrer du dioxyde de carbone hors d'un mélange de gaz
à l'aide d'un agencement d'absorption (100) qui comprend
- un absorbeur de CO2 (1),
- un piège à eau (2),
- une unité de guidage de fluide d'alimentation (3, 3.1, 3.2),
- une unité inférieure de guidage de fluide de déviation (9),
- une unité supérieure de guidage de fluide de déviation (6, 6.2, 33.b),
- une unité de guidage de fluide d'évacuation (34, 34.1) et
- une unité de guidage de fluide de liaison (33, 33.a) qui relie l'absorbeur de CO2 (1) au piège à eau (2),
dans lequel le procédé comprend les étapes selon lesquelles le mélange de gaz
- est dirigé depuis une source (31) jusqu'à l'unité de guidage de fluide d'alimentation (3, 3.1, 3.2),
- est dirigé à travers l'unité de guidage de fluide d'alimentation (3, 3.1, 3.2) jusqu'à l'unité inférieure de guidage de fluide de déviation (9),
- est dévié verticalement ou obliquement vers le haut par l'unité inférieure de guidage de fluide de déviation (9),
- s'écoule verticalement ou obliquement vers le haut à travers l'absorbeur de CO2 (1),
- est dévié verticalement ou obliquement vers le bas par l'unité supérieure de guidage de fluide de déviation (6, 6.2, 33.b),
- s'écoule verticalement ou obliquement vers le bas à travers l'unité de guidage de fluide de liaison (33, 33.a) 33.a) jusqu'au piège à eau (2),
- s'écoule à travers le piège à eau (2),
- s'écoule à travers l'unité de guidage de fluide d'évacuation (34, 34.1) et
- est dirigé depuis l'unité de guidage de fluide d'évacuation (34, 34.1) jusqu'à un collecteur (32),
dans lequel le procédé comprend l'étape supplémentaire selon laquelle
l'absorbeur de CO2 (1) absorbe le dioxyde de carbone pendant que le mélange de gaz s'écoule à travers l'absorbeur de CO2 (1), et filtre ainsi le dioxyde de carbone hors du mélange de gaz.
